(19) Europäisches Patentamt
European Patent Office
Office européen des brevets

(11) EP 0 822 830 B1

(12) EUROPEAN PATENT SPECIFICATION

(45) Date of publication and mention of the grant of the patent:
**02.04.2008 Bulletin 2008/14**

(21) Application number: **96913247.1**

(22) Date of filing: **29.04.1996**

(51) Int Cl.:
*A61K 39/00* (2006.01) *C07K 16/18* (2006.01)
*C07K 16/24* (2006.01) *C12N 15/13* (2006.01)
*C12N 5/16* (2006.01)

(86) International application number:
**PCT/US1996/005928**

(87) International publication number:
**WO 1996/033735 (31.10.1996 Gazette 1996/48)**

(54) **Human anti-IL-8 antibodies, derived from immunized xenomice**

Aus immunisierten Xenomäusen stammende menschliche Antikörper gegen IL-8

Anticorps anti-IL-8 dérivés de xenosouris immunisées

(84) Designated Contracting States:
**AT BE CH DE DK ES FI FR GB GR IE IT LI LU MC NL PT SE**

(30) Priority: **27.04.1995 US 430938**

(43) Date of publication of application:
**11.02.1998 Bulletin 1998/07**

(60) Divisional application:
**05028220.1 / 1 709 970**

(73) Proprietor: **Amgen Fremont Inc.**
**Fremont, CA 94555 (US)**

(72) Inventors:
- **KUCHERLAPATI, Raju**
**Darien, CT 06820 (US)**
- **JAKOBOVITS, Aya**
**Menlo Park, CA 94025 (US)**
- **KLAPHOLZ, Sue**
**Stanford, CA 94305 (US)**
- **BRENNER, Daniel, G.**
**Redwood City, CA 94601 (US)**
- **CAPON, Daniel, J.**
**Hillsborough, CA 94010 (US)**

(74) Representative: **Vossius & Partner**
**Postfach 86 07 67**
**81634 München (DE)**

(56) References cited:
**EP-A- 0 463 151**
**WO-A-91/10741**
**WO-A-92/03918**
**WO-A-93/12227**
**WO-A-94/02602**
**WO-A-94/25585**
**US-A- 5 286 647**

- **WINTER G ET AL: "MAKING ANTIBODIES BY PHAGE DISPLAY TECHNOLOGY" ANNUAL REVIEW OF IMMUNOLOGY, ANNUAL REVIEWS INC, US, vol. 12, 1994, pages 433-455, XP000564245 ISSN: 0732-0582**
- **LONBERG N ET AL: "HUMAN ANTIBODIES FROM TRANSGENIC MICE" INTERNATIONAL REVIEWS OF IMMUNOLOGY, HARWOOD ACADEMIC PUBLISHERS, LONDON, GB, vol. 13, 1995, pages 65-93, XP000944265 ISSN: 0883-0185**
- **LONBERG N ET AL: "ANTIGEN-SPECIFIC HUMAN ANTIBODIES FROM MICE COMPRISING FOUR DISTINCT GENETIC MODIFICATIONS" NATURE, MACMILLAN JOURNALS LTD. LONDON, GB, vol. 368, no. 6474, 28 April 1994 (1994-04-28), pages 856-859, XP000941636 ISSN: 0028-0836**
- **IKEMATSU H ET AL: "Clonal analysis of a human antibody response. II. Sequences of the VH genes of human IgM, IgG, IgA to rabies virus reveal preferential utilization of VHIII segments and somatic hypermutation" JOURNAL OF IMMUNOLOGY, THE WILLIAMS AND WILKINS CO. BALTIMORE, US, vol. 150, no. 4, 15 February 1993 (1993-02-15), pages 1325-1337, XP002197277 ISSN: 0022-1767**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- EXPERT OPINION ON INVESTIGATIONAL DRUGS, Volume 3, Number 3, issued March 1994, EMERY et al., "Humanised Monoclonal Antibodies for Therapeutic Applications", pages 241-251.
- ZACHAU: "The immunoglobulin k locus- or -what has been learned from looking closely at one thenth of a percent of the human genome" GENE, vol. 135, 1993, pages 167-173,
- COOK ET AL: "A map of the human immunoglobulin Vh locus completed by analysis of the telomeric region of chromosome 14q" NATURE GENETICS, vol. 7, 1994, pages 162-168,

## Description

### Technical Field

The invention relates to the field of immunology, and in particular to the production of antibodies. More specifically, it concerns producing anti-IL-8 antibodies by a process which includes the step of immunizing a transgenic animal with an antigen to which antibodies are desired. The transgenic animal has been modified so as to produce human, as opposed to endogenous, antibodies.

### Background Art

PCT application WO 94/02602, published 3 February 1994 describes in detail the production of transgenic nonhuman animals which are modified so as to produce fully human antibodies rather than endogenous antibodies in response to antigenic challenge. Briefly, the endogenous loci encoding the heavy and light immunoglobulin chains are incapacitated in the transgenic hosts and loci encoding human heavy and light chain proteins are inserted into the genome. In general, the animal which provides all the desired modifications is obtained by cross breeding intermediate animals containing fewer than the full complement of modifications. The preferred embodiment of nonhuman animal described in the specification is a mouse. Thus, mice, specifically, are described which, when administered immunogens, produce antibodies with human variable regions, including fully human antibodies, rather than murine antibodies that are immunospecific for these antigens.

The availability of such transgenic animals makes possible new approaches to the production of fully human antibodies. Antibodies with various immunospecificities are desirable for therapeutic and diagnostic use. Those antibodies intended for human therapeutic and *in vivo* diagnostic use, in particular, have been problematic because prior art sources for such antibodies resulted in immunoglobulins bearing the characteristic structures of antibodies produced by nonhuman hosts. Such antibodies tend to be immunogenic when used in humans.

The availability of the nonhuman, immunogen responsive transgenic animals described in the above-referenced WO 94/02602 make possible convenient production of human antibodies without the necessity of employing human hosts.

### Disclosure of the Invention

The invention is directed to anti-IL-8 antibodies as defined in the claims.

A method to produce a fully human immunoglobulin to a specific antigen or to produce an analog of said immunoglobulin by a process which comprises immunizing a nonhuman animal with the antigen under conditions that stimulate an immune response is disclosed. The nonhuman animal is characterized by being substantially incapable of producing endogenous heavy or light immunoglobulin chain, but capable of producing immunoglobulins with both human variable and constant regions. In the resulting immune response, the animal produces B cells which secrete immunoglobulins that are fully human and specific for the antigen. The human immunoglobulin of desired specificity can be directly recovered from the animal, for example, from the serum, or primary B cells can be obtained from the animal and immortalized. The immortalized B cells can be used directly as the source of human antibodies or, alternatively, the genes encoding the antibodies can be prepared from the immortalized B cells or from primary B cells of the blood or lymphoid tissue (spleen, tonsils, lymph nodes, bone marrow) of the immunized animal and expressed in recombinant hosts, with or without modifications, to produce the immunoglobulin or its analogs. In addition, the genes encoding the repertoire of immunoglobulins produced by the immunized animal can be used to generate a library of immunoglobulins to permit screening for those variable regions which provide the desired affinity. Clones from the library which have the desired characteristics can then be used as a source of nucleotide sequences encoding the desired variable regions for further manipulation to generate antibodies or analogs with these characteristics using standard recombinant techniques.

An immortalized nonhuman B cell line derived from the above described animal is disclosed. In still another aspect, the invention is directed to a recombinant host cell which is modified to contain the gene encoding the human immunoglobulin defined in the claims.

### Brief Description of the Drawings

Figure 1 is a schematic of the construction of the yH1C human heavy chain YAC.
Figure 2 is a schematic of the construction of the yK2 human kappa light chain YAC.
Figure 3 shows the serum titers of anti-IL-6 antibodies from a XenoMouse™ immunized with human IL-6 and which antibodies contain human κ light chains and/or human μ heavy chains.

Figure 4 show the serum titers of anti-TNFα antibodies from a XenoMouse™ immunized with human TNF-α and which antibodies contain human κ light chains and/or human μ heavy chains.

Figure 5 shows serum titers of anti-CD4 antibodies from a XenoMouse™ immunized with human CD4 and which antibodies contain human κ light chains and/or human μ heavy chains.

Figure 6 shows the serum titers of a XenoMouse™ immunized with 300.19 cells expressing L-selectin at their surface. In the ELISA assay used, these antibodies are detectable if they carry human μ constant region heavy chains.

Figure 7 shows the serum titers of a XenoMouse™ immunized with 300.19 cells expressing L-selectin at their surface. In the ELISA assay used, these antibodies are detectable only if they carry human κ light chains.

Figure 8 shows a FACS Analysis of human neutrophils incubated with serum from a XenoMouse™ immunized with human L-selectin and labeled with an antibody immunoreactive with human light chain κ region.

Figure 9 shows a diagram of a plasmid used to transfect mammalian cells to effect the production of the human protein gp39.

Figure 10 represents the serum titration curve of mice immunized with CHO cells expressing human gp39. The antibodies detected in this ELISA must be immunoreactive with gp39 and contain human heavy chain μ constant regions of human κ light chains.

Figure 11 is a titration curve with respect to monoclonal antibodies secreted by the hybridoma clone D5.1. This clone is obtained from a XenoMouse™ immunized with tetanus toxin C (TTC) and contains human κ light chain and human μ constant region in the heavy chain.

Figure 12 DNA sequence of the heavy chain of anti tetanus toxin monoclonal antibody D5.1.4 (a subclone of D5.1). Mutations form germline are boxed.

Figure 13 DNA sequence of the kappa light chain of anti-tetanus toxin monoclonal antibody D5.1.4. Mutations form germline are boxed.

Figure 14 shows the serum titers of anti-IL-8 antibodies of XenoMouse™ immunized with human IL-8 and which antibodies contain human κ light chains and/or human μ heavy chains.

Figure 15 Inhibition of IL-8 binding to human neutrophils by monoclonal anti-human-IL-8 antibodies.

Figure 16 (A-H) DNA sequences of the heavy chain and kappa light chain of the anti-IL-8 antibodies D1.1 (16A-B), K2.2 (16C-D), K4.2 (16E-F), and K4.3 (16G-H).

## Modes of Carrying Out the Invention

In general are disclosed, methods including administering an antigen for which human forms of immunospecific reagents are desired to a transgenic nonhuman animal which has been modified genetically so as to be capable of producing human, but not endogenous, antibodies. Typically, the animal has been modified to disable the endogenous heavy and/or kappa light chain loci in its genome, so that these endogenous loci are incapable of the rearrangement required to generate genes encoding immunoglobulins in response to an antigen. In addition, the animal will have been provided, stably, in its genome, at least one human heavy chain locus and at least one human light chain locus so that in response to an administered antigen, the human loci can rearrange to provide genes encoding human variable regions immunospecific for the antigen.

The details for constructing such an animal useful in the method of the invention are provided in the PCT application WO 94/02602 referenced above. Examples of YACs for the present invention can be found in, for example, Green et al. Nature Genetics 7:13-21 (1994). In a preferred embodiment of the XenoMouse™, the human heavy chain YAC, yH1C (1020 kb), and human light chain YAC, yK2 (880 kb) are used. yH1C is comprised of 870 kb of the human variable region, the entire D and $J_H$ region, human μ, δ, and γ2 constant regions and the mouse 3' enhancer. yK2 is comprised of 650 kb of the human kappa chain proximal variable region ($V_\kappa$), the entire $J_\kappa$ region, and $C_\kappa$ with its flanking sequences that contain the Kappa deleting element (κde). Both YACs also contain a human HPRT selectable marker on their YAC vector arm. Construction of yH1C and yK2 was accomplished by methods well known in the art. In brief, YAC clones bearing segments of the human immunoglobulin loci were identified by screening a YAC library (Calbertsen et al, PNAS 87:4256 (1990)) Overlapping clones were joined by recombination using standard techniques (Mendez et al. Genomics 26:294-307 (1995)). Details of the schemes for assembling yH1C and yK2 are shown in Figure 1 and Figure 2 respectively.

yK2 was constructed from the clones A80-C7, A210-F10 and A203-C6 from the Olson library, disclosed in, for example, Burke et al., Science 236:806-812 (1987), Brownstein et al., Science 244:1348-1351 (1989), and Burke et al., Methods in Enzymology 194:251-270 (1991).

For production of the desired antibodies, the first step is administration of the antigen. Techniques for such administration are conventional and involve suitable immunization protocols and formulations which will depend on the nature of the antigen per se. It may be necessary to provide the antigen with a carrier to enhance its immunogenicity and/or to include formulations which contain adjuvants and/or to administer multiple injections and/or to vary the route of the immunization, and the like. Such techniques are standard and optimization of them will depend on the characteristics of the particular antigen for which immunospecific reagents are desired.

As used herein, the term "immunospecific reagents" includes immunoglobulins and their analogs. The term "analogs" has a specific meaning in this context. It refers to moieties that contain the fully human portions of the immunoglobulin which account for its immunospecificity. In particular, complementarity determining regions (CDRs) are required, along with sufficient portions of the framework (Frs) to result in the appropriate three dimensional conformation. Typical immunospecific analogs of antibodies include $F(ab")_2$, Fab', and Fab regions. Modified forms of the variable regions to obtain, for example, single chain $F_v$ analogs with the appropriate immunospecificity are known. A review of such $F_v$ construction is found, for example, in Huston et al., Methods in Enzymology 203:46-63 (1991). The construction of antibody analogs with multiple immunospecificities is also possible by coupling the variable regions from one antibody to those of second antibody.

The variable regions with fully human characteristics can also be coupled to a variety of additional substances which can provide toxicity, biological functionality, alternative binding specificities and the like. The moieties including the fully human variable regions produced by the methods of the invention include single-chain fusion proteins, molecules coupled by covalent methods other than those involving peptide linkages, and aggregated molecules. Examples of analogs which include variable regions coupled to additional molecules covalently or noncovalently include those in the following nonlimiting illustrative list. Traunecker, A. et al. Int. J. Cancer Supp (1992) Supp 7:51-52 describe the bispecific reagent janusin in which the $F_v$ region directed to CD3 is coupled to soluble CD4 or to other ligands such as OVCA and IL-7. Similarly, the fully human variable regions produced by the method of the invention can be constructed into $F_v$ molecules and coupled to alternative ligands such as those illustrated in the cited article. Higgins, P.J. et al J. Infect Disease (1992) 166:198-202 described a heteroconjugate antibody composed of OKT3 cross-linked to an antibody directed to a specific sequence in the V3 region of GP120. Such heteroconjugate antibodies can also be constructed using at least the human variable regions contained in the immunoglobulins produced by the invention methods. Additional examples of bispecific antibodies include those described by Fanger, M.W. et al. Cancer Treat Res (1993) 68:181-194 and by Fanger, M.W. et al. Crit Rev Immunol (1992) 12:101-124. Conjugates that are immunotoxins including conventional antibodies have been widely described in the art. The toxins may be coupled to the antibodies by conventional coupling techniques or immunotoxins containing protein toxin portions can be produced as fusion proteins. The analogs of the present invention can be used in a corresponding way to obtain such immunotoxins. Illustrative of such immunotoxins are those described by Byers, B.S. et al. Seminars Cell Biol (1991) 2:59-70 and by Fanger, M.W. et al. Immunol Today (1991) 12:51-54.

Immunoglobulins and analogs can have agonist activity with respect to antigens for which they are immunospecific in the cases wherein the antigens perform signal transducing functions. Thus, a subset of antibodies or analogs prepared according to the disclosed methods which are immunospecific for, for example, a cell surface receptor, will be capable of eliciting a response from cells bearing this receptor corresponding to that elicited by the native ligand. Furthermore, antibodies or analogs which are immunospecific for substances mimicking transition states of chemical reactions will have catalytic activity.

In short, the genes encoding the immunoglobulins produced by the transgenic animals of the invention can be retrieved and the nucleotide sequences encoding the fully human variable region can be manipulated according to known techniques to provide a variety of analogs such as those described above. In addition, the immunoglobulins themselves containing the human variable regions can be modified using standard coupling techniques to provide conjugates retaining immunospecific regions.

Thus, immunoglobulin "analogs" refers to the moieties which contain those portions of the antibodies of the invention which retain their human characteristics and their immunospecificity. These will retain sufficient human variable regions to provide the desired specificity.

Methods including administering the appropriate antigen to the transgenic animal are disclosed. The recovery or production of the antibodies themselves can be achieved in various ways.

First, and most straightforward, the polyclonal antibodies produced by the animal and secreted into the bloodstream can be recovered using known techniques. Purified forms of these antibodies can, of course, be readily prepared by standard purification techniques, preferably including affinity chromatography with Protein A, anti-immunoglobulin, or the antigen itself. In any case, in order to monitor the success of immunization, the antibody levels with respect to the antigen in serum will be monitored using standard techniques such as ELISA, RIA and the like.

For some applications only the variable regions of the antibodies are required. Treating the polyclonal antiserum with suitable reagents so as to generate Fab', Fab, or $F(ab")_2$ portions results in compositions retaining fully human characteristics. Such fragments are sufficient for use, for example, in immunodiagnostic procedures involving coupling the immunospecific portions of immunoglobulins to detecting reagents such as radioisotopes.

Alternatively, immunoglobulins and analogs with desired characteristics can be generated from immortalized B cells derived from the transgenic animals used in the method of the invention or from the rearranged genes provided by these animals in response to immunization.

Thus, as an alternative to harvesting the antibodies directly from the animal, the B cells can be obtained, typically from the spleen, but also, if desired, from the peripheral blood lymphocytes or lymph nodes and immortalized using any of a variety of techniques, most commonly using the fusion methods described by Kohler and Milstein Nature 245:495 (1975) The resulting hybridomas (or otherwise immortalized B cells) can then be cultured as single colonies and screened for secretion of antibodies of the desired specificity. As described above, the screen can also include a confirmation of the fully human character of the antibody. For example, as described in the examples below, a sandwich ELISA wherein the monoclonal in the hybridoma supernatant is bound both to antigen and to an antihuman constant region can be employed. After the appropriate hybridomas are selected, the desired antibodies can be recovered, again using conventional techniques. They can be prepared in quantity by culturing the immortalized B cells using conventional methods, either in vitro or in vivo to produce ascites fluid. Purification of the resulting monoclonal antibody preparations is less burdensome that in the case of serum since each immortalized colony will secrete only a single type of antibody. In any event, standard purification techniques to isolate the antibody from other proteins in the culture medium can be employed.

As an alternative to obtaining human immunoglobulins directly from the culture of immortalized B cells derived from the animal, the immortalized cells can be used as a source of rearranged heavy chain and light chain loci for subsequent expression and/or genetic manipulation. Rearranged antibody genes can be reverse transcribed from appropriate mRNAs to produce cDNA. If desired, the heavy chain constant region can be exchanged for that of a different isotype or eliminated altogether. The variable regions can be linked to encode single chain $F_v$ regions. Multiple $F_v$ regions can be linked to confer binding ability to more than one target or chimeric heavy and light chain combinations can be employed. Once the genetic material is available, design of analogs as described above which retain both their ability to bind the desired target, and their human characteristics, is straightforward.

Once the appropriate genetic material is obtained and, if desired, modified to encode an analog, the coding sequences, including those that encode, at a minimum, the variable regions of the human heavy and light chain, can be inserted into expression systems contained on vectors which can be transfected into standard recombinant host cells. As described below, a variety of such host cells may be used; for efficient processing, however, mammalian cells are preferred. Typical mammalian cell lines useful for this purpose include CHO cells, 293 cells, or NSO cells.

The production of the antibody or analog is then undertaken by culturing the modified recombinant host under culture conditions appropriate for the growth of the host cells and the expression of the coding sequences. The antibodies are then recovered from the culture. The expression systems are preferably designed to include signal peptides so that the resulting antibodies are secreted into the medium; however, intracellular production is also possible.

In addition to deliberate design of modified forms of the immunoglobulin genes to produce analogs, advantage can be taken of phage display techniques to provide libraries containing a repertoire of antibodies with varying affinities for the desired antigen. For production of such repertoires, it is unnecessary to immortalize the B cells from the immunized animal; rather, the primary B cells can be used directly as a source of DNA. The mixture of cDNAs obtained from B cells, e.g., derived from spleens, is used to prepare an expression library, for example, a phage display library transfected into *E. coli.* The resulting cells are tested for immunoreactivity to the desired antigen. Techniques for the identification of high affinity human antibodies from such libraries are described by Griffiths, A.D., et al., EMBO J (1994) 13:3245-3260; by Nissim, A., et al. ibid, 692-698, and by Griffiths, A.D., et al., ibid, 12:725-734. Ultimately, clones from the library are identified which produce binding affinities of a desired magnitude for the antigen, and the DNA encoding the product responsible for such binding is recovered and manipulated for standard recombinant expression. Phage display libraries may also be constructed using previously manipulated nucleotide sequences and screened in similar fashion. In general, the cDNAs encoding heavy and light chain are independently supplied or are linked to form $F_v$ analogs for production in the phage library.

The phage library is then screened for the antibodies with highest affinity for the antigen and the genetic material recovered from the appropriate clone. Further rounds of screening can increase the affinity of the original antibody isolated. The manipulations described above for recombinant production of the antibody or modification to form a desired analog can then be employed.

Combination of phage display technology with the XenoMouse™ offers a significant advantage over previous applications of phage display. Typically, to generate a highly human antibody by phage display, a combinatorial antibody library is prepared either from human bone marrow or from peripheral blood lymphocytes as described by Burton, D.R., et al., Proc. Natl. Acad. Sci. USA (1991) 88:10134-10137. Using this approach, it has been possible to isolate high affinity antibodies to human pathogens from infected individuals, i.e. from individuals who have been "immunized" as described in Burton, D.R., et al., Proc. Natl. Acad. Sci. USA (1991) 88:10134-10137, Zebedee, S.L., et al. Proc. Natl. Acad. Sci. USA (1992) 89:3175-3179, and Barbas III, C.F., et al., Proc. Natl. Acad. Sci. USA (1991) 89:10164-20168. However, to generate antibodies reactive with human antigens, it has been necessary to generate synthetic libraries (Barbas III C.F., et al., Proc. Natl. Acad. Sci. USA (1991) 89:4457-4461, Crameri, A. et al., BioTechniques (1995) 88: 194-196) or to prepare libraries from either autoimmune patients (Rapoport, B., et al., Immunol. Today (1995) 16:43-49, Portolano, S., et al., J. Immunol. (1993) 151:2839-2851, and Vogel, M., et al., Eur J. Immunol. (1994) 24:1200-1207) or normal individuals, i.e. naive libraries (Griffiths, A.D., et al., EMBO J. (1994) 13:3245-3260, Griffiths, A.D., et al., EMBO J. (1993) 12:725-734, Persson, M.A.A., et al., Proc. Natl. Acad. Sci. USA (1991) 88:2432-2436, Griffiths, A.D., Curr. Opin. Immunol. (1993) 5:263-267, Hoogenboom, H.R., et al., J. Mol. Biol. (1992) 227:381-388, Lerner, R.A., et al., Science (1992) 258:1313-1314, and Nissim A., et al., EMBO J. (1994) 13:692-698. Typically, high affinity antibodies to human proteins have proven very difficult to isolate in this way. As is well known, affinity maturation requires somatic mutation and somatic mutation, in turn, is antigen driven. In the XenoMouse, repeated immunization with human proteins will lead to somatic mutation and, consequently, high affinity antibodies. The genes encoding these antibodies can be readily amplified by PCR as described in Marks, J.D., et al., J. Mol. Biol. (1991) 581-596 and immunospecific antibodies isolated by standard panning techniques, Winter, G., et al., Annu. Rev. Immunol. (1994) 12:433-55 and Barbas III, C.F., et al., Proc. Natl. Acad. Sci. USA (1991) 88:7978-7982.

As above, the modified or unmodified rearranged loci are manipulated using standard recombinant techniques by constructing expression systems operable in a desired host cell, such as, typically, a Chinese hamster ovary cell, and the desired immunoglobulin or analog is produced using standard recombinant expression techniques, and recovered and purified using conventional methods.

The application of the foregoing processes to antibody production has enabled the preparation of human immunospecific reagents with respect to antigens for which human antibodies have not heretofore been available. The immunoglobulins that result from the above-described methods and the analogs made possible thereby provide novel compositions for use in analysis, diagnosis, research, and therapy. The particular use will, of course, depend on the immunoglobulin or analog prepared. In general, the compositions of the invention will have utilities similar to those ascribable to nonhuman antibodies directed against the same antigen. Such utilities include, for example, use as affinity ligands for purification, as reagents in immunoassays, as components of immunoconjugates, and as therapeutic agents for appropriate indications.

Particularly in the case of therapeutic agents or diagnostic agents for use in vivo, it is highly advantageous to employ antibodies or their analogs with fully human characteristics. These reagents avoid the undesired immune responses engendered by antibodies or analogs which have characteristics marking them as originating from nonhuman species. Other attempts to "humanize" antibodies do not result in reagents with fully human characteristics. For example, chimeric antibodies with murine variable regions and human constant regions are easily prepared, but, of course, retain murine characteristics in the variable regions. Even the much more difficult procedure of "humanizing" the variable regions by manipulating the genes encoding the amino acid sequences that form the framework regions does not provide the desired result since the CDRs, typically of nonhuman origin, cannot be manipulated without destroying immunospecificity.

Human antibodies against IL-8 may be used for the treatment or prevention of a pathology or condition associated with IL-8.

Moore et al., Nature, vol. 365, (14. 10. 1993) discloses non-human anti-IL-8 antibodies preventing lung reperfusion injury.

WO 92/04372 discloses mouse antibodies against IL-8 peptides. Polyclonal antibodies were shown to inhibit neutrophil activation.

Such conditions include, but are not limited to, tumor metastasis, reperfusion injury, pulmonary edema, asthma, ischemic disease such as myocardial infarction,inflammatory bowel disease (such as Crohn's disease and ulcerative colitis), encephalitis, uveitis, autoimmune diseases (such as rheumatoid arthritis, Sjögren's syndrome, vasculitis), osteoarthritis, gouty arthritis, nephritis, renal failure, dermatological conditions such as inflammatory dermatitis, psoriasis, vasculitic urticaria and allergic angiitis, retinal uveitis, conjunctivitis, neurological disorders such as stroke, multiple sclerosis and meningitis, acute lung injury, adult respiratory distress syndrome (ARDS), septic shock, bacterial pneumonia, diseases involving leukocyte diapedesis, CNS inflammatory disorder, multiple organ failure, alcoholic hepatitis, antigen-antibody complex mediated diseases, inflammation of the lung (such as pleurisy, aveolitis, vasculitis, pneumonia, chronic bronchitis, bronchiectasis, cystic fibrosis), Behcet disease, Wegener's granulomatosis, and vasculitic syndrome.

Typical autoimmune diseases which can be treated using the above-mentioned antibodies and analogs include systemic lupus erythematosus, rheumatoid arthritis, psoriasis, Sjogren's scleroderma, mixed connective tissue disease, dermatomyositis, polymyositis, Reiter's syndrome, Behcet's disease, Type 1 diabetes, Hashimoto's thyroiditis, Grave's disease, multiple sclerosis, myasthenia gravis and pemphigus.

For therapeutic applications, the antibodies may be administered in a pharmaceutically acceptable dosage form. They may be administered by any means that enables the active agent to reach the desired site of action, for example, intravenously as by bolus or by continuous infusion over a period of time, by intramuscular, subcutaneous, intraarticular, intrasynovial, intrathecal, oral, topical or inhalation routes. The antibodies may be administered as a single dose or a series of treatments.

For parenteral administration, the antibodies may be formulated as a solution, suspension, emulsion or lyophilized powder in association with a pharmaceutically acceptable parenteral vehicle. If the antibody is suitable for oral administration, the formulation may contain suitable additives such as, for example, starch, cellulose, silica, various sugars, magnesium carbonate, or calcium phosphate. Suitable vehicles are described in the most recent edition of Remington's Pharmaceutical Sciences, A. Osol, a standard reference text in this field.

For prevention or treatment of disease, the appropriate dosage of antibody will depend upon known factors such as the pharmacodynamic characteristics of the particular antibody, its mode and route of administration, the age, weight, and health of the recipient, the type of condition to be treated and the severity and course of the condition, frequency of treatment, concurrent treatment and the physiological effect desired. The examples below are intended to illustrate but not to limit the invention.

In these examples, mice, designated XenoMouse™, are used for initial immunizations. A detailed description of the XenoMouse™ is found in the above referenced PCT application WO 94/02602. Immunization protocols appropriate to each antigen are described in the specific examples below. The sera of the immunized XenoMouse™ (or the supernatants from immortalized B cells) were titrated for antigen specific human antibodies in each case using a standard ELISA format. In this format, the antigen used for immunization was immobilized onto wells of microtiter plates. The plates were washed and blocked and the sera (or supernatants) were added as serial dilutions for 1-2 hours of incubation. After washing, bound antibody having human characteristics was detected by adding antihuman κ, μ, or γ chain antibody conjugated to horseradish peroxidase (HRP) for one hour. After again washing, the chromogenic reagent o-phenylene diamine (OPD) substrate and hydrogen peroxide were added and the plates were read 30 minutes later at 492 nm using a microplate reader.

Unless otherwise noted, the antigen was coated using plate coating buffer (0.1 M carbonate buffer, pH 9.6); the assay blocking buffer used was 0.5% BSA, 0.1% Tween 20 and 0.01% thimerosal in PBS; the substrate buffer used in color development was citric acid 7.14 g/l; dibasic sodium phosphate 17.96 g/l; the developing solution (made immediately before use) was 10 ml substrate buffer; 10 mg OPD, plus 5 ml hydrogen peroxide; the stop solution (used to stop color development) was 2 M sulfuric acid. The wash solution was 0.05% Tween 20 in PBS.

<u>Example 1</u>

<u>Human Antibodies Against Human IL-6</u>

Three to five XenoMouse™ aged 8-20 weeks were age-matched and immunized intraperitoneally with 50 μg human IL-6 emulsified in incomplete Freund's adjuvant for primary immunization and in complete Freund's adjuvant for subsequent injections. The mice received 6 injections 2-3 weeks apart. Serum titers were determined after the second dose and following each dose thereafter. Bleeds were performed from the retrobulbar plexus 6-7 days after injections. The blood was allowed to clot at room temperature for about 2 hours and then incubated at 4˚C for at least 2 hours before separating and collecting the sera.

ELISAs were conducted as described above by applying 100 μl/well of recombinant human IL-6 at 2 μg/ml in coating buffer. Plates were then incubated at 4˚C overnight or at 37˚C for 2 hours and then washed three times in washing buffer. Addition of 100 μl/well blocking buffer was followed by incubation at room temperature for 2 hours, and an additional 3 washes.

Then, 50 μl/well of diluted serum samples (and positive and negative controls) were added to the plates. Plates were then incubated at room temperature for 2 hours and again washed 3 times.

After washing, 100 μl/well of either mouse antihuman μ chain antibody conjugated to HRP at 1/2,000 or mouse antihuman κ chain antibody conjugated to HRP at 1/2,000, diluted in blocking buffer was added. After a 1 hour incubation at room temperature, the plates were washed 3 times and developed with OPD substrate for 10-25 minutes. 50 μl/well of stop solution was then added and the results read on an ELISA plate reader at 492 nm. The dilution curves resulting from the titration of serum from XenoMouse™ after 6 injections are shown in Figure 3. The data in Figure 3 show production of anti-IL-6 immunoreactive with antihuman κ and antihuman μ detectable at serum dilutions above 1:1,000.

<u>Example 2</u>

<u>Human Antibodies Against Human TNFα</u>

Immunization and serum preparation were conducted as described in Example 1 except that human recombinant TNFα (at 5μg per injection) was substituted for human IL-6. ELISAs were conducted as described in Example 1 except that the initial coating of the ELISA plate employed 100 μl/well recombinant human TNFα at 1 μg/ml in coating buffer.

The dilution curves for serum from XenoMouse™ after 6 inductions obtained are shown in Figure 4. Again significant titers of human anti-TNFα binding were shown.

Serum titers for hγ, hμ, and hκ after one and two immunizations of the XenoMouse™ are shown in Table 1. When challenged with TNF-α, the XenoMouse™ switches isotypes from a predominant IgM response in the first immunization to an immune response with a large IgG component in the second immunization.

TABLE 2. Anti TNF-alpha serum titer responses of Xenomouse-2.

| Bleed 1: after 2 immunizations<br>Bleed 2: after 3 immunizations | | | | |
|---|---|---|---|---|
| XM2 | | ELISA Serum titers Specific for TNF-alpha | | |
| | | titer (via hγ) | titer (via hμ) | titer (via hκ) |
| 1 | bleed 1 | 500 | 3,000 | 1,500 |
| | bleed 2 | 10,000 | 8,000 | 15,000 |
| 2 | bleed 1 | 200 | 3,000 | 500 |
| | bleed 2 | 2,700 | 5,000 | 1,000 |
| 3 | bleed 1 | <500 | 2,000 | 1,500 |
| | bleed 2 | 15,000 | 24,000 | 25,000 |
| 4 | bleed 1 | 500 | 2,500 | 1,500 |
| | bleed 2 | 70,000 | 4,000 | 72,000 |
| 5 | bleed 1 | <500 | 2,500 | 1,500 |
| | bleed 2 | 1,000 | 10,000 | 7,000 |
| 6 | bleed 1 | 1,000 | 13,000 | 4,500 |
| | bleed 2 | 10,000 | 24,000 | 25.000 |
| 7 | bleed 1 | <500 | 2,500 | 1,500 |
| | bleed 2 | 5,000 | 4,000 | 9,000 |
| 8 | bleed 1 | <500 | 1,000 | 500 |
| | bleed 2 | 2,700 | 5,000 | 9,000 |
| 9 | bleed 1 | 200 | 6,000 | 4,000 |
| | bleed 2 | 40,000 | 80,000 | 80,000 |
| 10 | bleed 1 | 200 | 2,000 | 500 |
| | bleed 2 | 15,000 | 8,000 | 60,000 |
| 11 | bleed 1 | 1,500 | 1,000 | 1,500 |
| | bleed 2 | 24,000 | 2,700 | 72,000 |
| 12 | bleed 1 | 200 | 2,000 | 1,000 |
| | bleed 2 | 10,000 | 4,000 | 25,000 |
| 13 | bleed 1 | 500 | 30,000 | 500 |
| | bleed 2 | 2,000 | 4,000 | 12,000 |

Example 3

Human antibodies Against Human CD4

The human CD4 antigen was prepared as a surface protein using human CD4ζ on transfected recombinant cells as follows. Human CD4ζ consists of the extracellular domain of CD4, the transmembrane domain of CD4, and the cytoplasmic domain corresponding to residues 31-142, of the mature ζ chain of the CD3 complex. Human CD4 zeta (F15 LTR) as described in Roberts et al., Blood (1994) 84:2878 was introduced into the rat basophil leukemic cell line RBL-2H3, described by Callan, M., et al., Proc Natl Acad Sci USA (1993) 90:10454 using the Kat high efficiency transduction described by Finer et al., Blood (1994) 83:43. Briefly, RBL-2H3 cells at $10^6$ cells per well were cultured in 750 μl DMEM$^{1∞}$ + 20% FBS (Gibco) and 16 μg/ml polybrene with an equal volume of proviral supernatant for 2 hours at 37˚C, 5% $CO_2$. One ml of medium was removed and 750 μl of infection medium and retroviral supernatant were added to each well and the cultures incubated overnight. The cells were washed and expanded in DMEM$^{1∞}$ + 10% FBS until sufficient cells were available for sorting. The CD4 zeta transduced RBL-2H3 cells were sorted using the FACSTAR plus (Becton Dickinson). The cells were stained for human CD4 with a mouse antihuman CD4 PE antibody and the top 2-3% expressing cells were selected.

Immunizations were conducted as described in Example 1 using 1 X $10^7$ cells per mouse except that the primary injection was subcutaneous at the base of the neck. The mice received 6 injections 2-3 weeks apart. Serum was prepared and analyzed by ELISA as described in Example 1 except that the initial coating of the ELISA plate utilized 100 μl per well of recombinant soluble CD4 at 2 μg/ml of coating buffer. The titration curve for serum from XenoMouse™ after 6 injections is shown in Figure 5. Titers of human anti-CD4 reactivity were shown at concentrations representing greater than those of 1:1,000 dilution.

Example 4

Human Antibodies Against Human L-selectin

The antigen was prepared as a surface displayed protein in C51 cells, a high expressing clone derived by transfecting the mouse pre-B cell 300.19 with LAM-1 cDNA (LAM-1 is the gene encoding L-selectin) (Tedder, et al., J. Immunol (1990) 144:532) or with similarly transfected CHO cells. The transfected cells were sorted using fluorescent activated cell sorting using anti-Leu-8 antibody as label.

The C51 and the transfected CHO cells were grown in DME 4.5 g/l glucose with 10% FCS and 1 mg/ml G418 in 100 mm dishes. Negative control cells, 3T3-P317 (transfected with gag/pol/env genes of Moloney virus) were grown in the same medium without G418.

Primary immunization was done by injection subcutaneously at the base of the neck; subsequent injections were intraperitoneal. 70-100 million C51 or transfected CHO cells were used per injection for a total of five injections 2-3 weeks apart.

Sera were collected as described in Example 1 and analyzed by ELISA in a protocol similar to that set forth in Example 1.

For the ELISA, the transfected cells were plated into 96 well plates and cell monolayers grown for 1-2 days depending on cell number and used for ELISA when confluent. The cells were fixed by first washing with cold 1 x PBS and then fixing solution (5% glacial acetic acid, 95% ethanol) was added. The plates were incubated at -25°C for 5 minutes and can be stored at this temperature if sealed with plate sealers.

The ELISA is begun by bringing the plates to room temperature, flicking to remove fixing solution and washing 5 times with DMEM medium containing 10% FCS at 200 μl per well.

The wells were treated with various serum dilutions or with positive or negative controls. Positive control wells contained murine IgGl monoclonal antibody to human L-selectin.

The wells were incubated for 45 minutes and monolayer integrity was checked under a microscope. The wells were then incubated with antihuman κ chain antibody or antihuman μ chain antibody conjugates with HRP described in Example 1. The plates were then washed with 1% BSA/PBS and again with PBS and monolayer integrity was checked. The plates were developed, stopped, and read as described above. The results for serum from XenoMouse™ are shown in Figures 6 and 7; human antibodies both to L-selectin and control 3T3 cells were obtained. However, the serum titers are higher for the L-selectin-expressing cells as compared to parental 3T3 cells. These results show the XenoMouse™ produces antibodies specific for L-selectin with human μ heavy chain regions and human κ light chains.

The antisera obtained from the immunized XenoMouse™ were also tested for staining of human neutrophils which express L-selectin. Human neutrophils were prepared as follows:

peripheral blood was collected from normal volunteers with 100 units/ml heparin. About 3.5 ml blood was layered over an equal volume of One-step Polymorph Gradient (Accurate Chemical, Westbury, NY) and spun for 30 minutes at 450 x g at 20°C. The neutrophil fraction was removed and washed twice in DPBS/2% FBS.

The neutrophils were then stained with either;

(1) antiserum from XenoMouse™ immunized with C51 cells (expressing L-selectin);
(2) as a negative control, antiserum from a XenoMouse™ immunized with cells expressing human gp39.

The stained, washed neutrophils were analyzed by FACS. The results for antiserum from XenoMouse™ are shown in Figure 8.

These results show the presence of antibodies in immunized XenoMouse™ serum which contain fully human light chains immunoreactive with L-selectin. The negative control antiserum from mice immunized with gp39 does not contain antibodies reactive against human neutrophils.

Example 5

Human Antibodies Against Human gp39

gp39 (the ligand for CD40) is expressed on activated human CD4 T cells. The sera of XenoMouse™ immunized with recombinant gp39 according to this example contained fully human antibodies immunospecific for gp39.
The antigen consisted of stable transfectants of 300.19 cells or of CHO cells expressing gp39 cDNA cloned into the mammalian expression vector P1K1.HUgp39/IRES NEO as shown in Figure 9. CHO cells were split 1:10 prior to transfection in DMEM 4.5 g/l glucose, 10% FBS, 2 mM glutamine, MEM, NEAA supplemented with additional glycine, hypoxanthine and thymidine. The cells were cotransfected with the gp39 vector at 9 μg/10 cm plate (6 x $10^5$ cells) and the DHFR expressing vector pSV2DHFRs (Subranani et al., Mol Cell Biol (1981) 9:854) at 1 μg/10 cm plate using calcium phosphate transfection. 24 hours later the cells were split 1:10 into the original medium containing G418 at 0.6 mg/ml. Cells producing gp39 were sorted by FACS using an anti-gp39 antibody.
Mice grouped as described in Example 1 were immunized with 300.19 cells expressing gp39 using primary immunization subcutaneously at the base of the neck and with secondary intraperitoneal injections every 2-3 weeks. Sera were harvested as described in Example 1 for the ELISA assay. The ELISA procedure was conducted substantially as set forth in Example 1; the microtiter plates were coated with CHO cells expressing gp39 grown in a 100 mm dish in DMEM, 4.5 g/l glucose, 10% FCS, 4mM glutamine, and nonessential amino acid (NEAA) solution for MEM (100X). On the day preceding the ELISA assay, the cells were trypsinized and plated into well filtration plates at $10^5$ cells/200 μl well and incubated at 37°C overnight. The positive controls were mouse antihuman gp39; negative controls were antisera from mice immunized with an antigen other than gp39. 50 μl of sample were used for each assay. The remainder of the assay is as described in Example 1.
The dilution curves for the sera obtained after 4 injections from mice immunized with gp39 expressed on CHO cells are shown in Figure 10. As shown, the sera contained antihuman gp39 immunospecificity which is detectable with anti-human κ and anti-human μ chain antibodies coupled to HRP.

Example 6

Preparation of Human Mabs Against Tetanus Toxin

The antibodies prepared in this example were secreted by hybridomas obtained by immortalizing B cells from xenomice immunized with tetanus toxin. The immunization protocol was similar to that set forth in Example 1 using 50 μg tetanus toxin emulsified in complete Freund's adjuvant for intraperitoneal primary immunization followed by subsequent intraperitoneal injections with antigen incorporated into incomplete Freund's adjuvant. The mice received a total of 4 injections 2-3 weeks apart.
After acceptable serum titers of antitetanus toxin C (anti-TTC) were obtained, a final immunization dose of antigen in PBS was give 4 days before the animals were sacrificed and the spleens were harvested for fusion.
The spleen cells were fused with myeloma cells P3X63-Ag8.653 as described by Galfre, G. and Milstein, C. Methods in Enzymology (1981) 73:3-46.
After fusion the cells were resuspended in DMEM, 15% FCS, containing HAT supplemented with glutamine, pen/strep for culture at 37°C and 10% $CO_2$. The cells were plated in microtiter plates and maintained in HAT-supplemented medium for two weeks before transfer to HAT-supplemented medium. Supernatants from wells containing hybridomas were collected for a primary screen using an ELISA.
The ELISA was conducted as described in Example 1 wherein the antigen coating consisted of 100 μl/well of tetanus toxin *C* (TTC) protein at 2 μg/ml in coating buffer, followed by incubation at 4°C overnight or at 37°C for two hours. In the primary ELISA, HRP-conjugated mouse antihuman IgM was used as described in Example 1. Two hybridomas that secreted anti-TTC according to the ELISA assay, clone D5.1 and clone K4.1 were used for further analysis.
As shown in Figure 11, clone D5.1 secretes fully human anti-TTC which is detectable using HRP-conjugated antihuman μ chain antibody and HRP-conjugated antihuman κ chain antibody. This is confirmed in Figure 11.
The antibody secreted by D5.1 did not immunoreact in ELISAs using TNFα, IL-6, or IL-8 as immobilized antigen under conditions where positive controls (sera from xenomice immunized with TNFα, IL-6 and IL-8 respectively) showed positive ELISA results.
The complete nucleotide sequence of the cDNAs encoding the heavy and light chains of the monoclonal were determined as shown in Figures 12 and 13. polyA mRNA was isolated from about $10^6$ hybridoma cells and used to generate cDNA using random hexamers as primers. Portions of the product were amplified by PCR using the appropriate primers.
The cell line was known to provide human κ light chains; for PCR amplification of light chain encoding cDNA, the primers used were HKPl (5'-CTCTGTGACACTCTCCTGGGAGTT-3') for priming from the constant region terminus and two oligos, used in equal amounts to prime from the variable segments; B3 (5'-GAAACGACACTCACGCAGTCTCCAGC-3').

For amplification of the heavy chain of the antibody derived form D5.1 (which contains the human μ constant region), MG-24VI was used to prime from the variable and μP1 (5'-TTTTCTTTGTTGCCGTTGGGGTGC-3') was used to prime from the constant region terminus.

Referring to Figure 12 which sets forth the sequence for the heavy chain of the antibody secreted by clone D5.1, this shows the heavy chain is comprised of the human variable fragment VH6, the human diversity region DN1 and the human joining segment JH4 linked to the human μ constant region. There were two base-pair mutations from the germline sequence in the variable region, both in the CDRs. Two additional mutations were in the D segment and six nongermline nucleotide additions were present at the $D_b$-$J_b$ junction.

Finally, referring to Figure 13 which presents the light chain of the antibody secreted by D5.1, the human κ variable region B3 and human κ joining region JK3 are shown. There are nine base-pair differences from the germline sequences, three falling with CDR1.

Example 7

Human Antibodies Against PTHrp

Groups of XenoMouse™-2 were immunized intraperitoneally with either PTHrp (1-34) conjugated with BTG, as described by Ratcliffe et al., J. Immunol. Methods 127:109 (1990), or with PTHrp (1-34) synthesized as a 4 branched-MAP (multiple antigenic peptide system). The antigens were emulsified in CFA (complete Freunds adjuvant) and injected i.p. at a dose of 25 μg per animal at 2 week intervals, and bled after two injections. The sera obtained from this bleed were analyzed by ELISA as described supra.

Serum titers for hγ, hμ, and hκ after one immunization of the XenoMouse™ are shown in Table 2. When immunized with PTHrp, the XenoMouse™ showed low serum titers in 5 of 7 mice on the first bleed, but when PTHrp-MAP is used, 7 of 7 mice show high serum titers on the first bleed.

TABLE 1. AntiPTHrp serum titer responses of Xenomouse-2.

| First bleed after 2 immunizations with either PTHrp-BTG conjugate | | | |
|---|---|---|---|
| **XM2 PTHrp-BTG Conjugate** | **Human Responses** | | |
| | **titer (via hγ)** | **titer (via hμ)** | **titer (via hκ)** |
| 1 | <30 | 850 | 100 |
| 2 | <30 | 3.000 | 50 |
| 3 | <30 | 7.000 | 1.000 |
| 4 | <30 | 800 | 200 |
| 5 | <30 | 400 | 90 |
| 6 | <30 | 500 | 50 |
| 7 | <30 | 300 | 50 |
| **XM2 PTHrp-MAP** | **titer (via hγ)** | **titer (via hμ)** | **titer (via hκ)** |
| 1 | <30 | 1,000 | 50 |
| 2 | <30 | 2,500 | 300 |
| 3 | <30 | 1,200 | 150 |
| 4 | 150 | 1,000 | 270 |
| 5 | 100 | 2,500 | 300 |
| 6 | <30 | 1,000 | 150 |
| 7 | <30 | 4,000 | 800 |

Example 8

Human Antibodies Against Human IL-8

Immunization and serum preparation were as described in Example 1 except that human recombinant IL-8 was used as an immunogen.

ELISA assays were performed with respect to the recovered serum, also exactly as described in Example 1, except that the ELISA plates were initially coated using 100 μl/well of recombinant human IL-8 at 0.5mg/ml in the coating buffer. The results obtained for various serum dilutions from XenoMouse™ after 6 injections are shown in Figure 14. Human anti-IL-8 binding was again shown at serum dilutions having concentrations higher than that represented by a 1:1,000 dilution.

Example 9

Preparation of High Affinity Human Monoclonal Antibodies Against Human IL-8

Groups of 4 to 6 XenoMouse™ aged between 8 to 10 weeks old were used for immunization and for hybridoma generation. XenoMouse™ were immunized intraperitoneally with 25 μg of human recombinant-IL-8 (Biosource International, CA, USA) emulsified in complete Freund's adjuvant (CFA, Sigma) for the primary immunization. All subsequent injections were done with the antigen incorporated into incomplete Freund's adjuvant (IFA, Sigma). For animals used as spleen donors for hybridoma generation a final dose of antigen in phosphate buffer saline (PBS) was given 4 days before the fusion. Serum titers of immunized XenoMouse™ were first analyzed after a secondary dose of antigens, and from there after, following every antigen dose. Test bleeds were performed 6 to 7 days after the injections, by bleeding from the retrobulbar plexus. Blood was allowed to clot at room temperature for about 2 hours and then incubated at 4°C for at least 2 hours before separating and collecting the sera.

Generation of hybridomas

Spleen cells obtained from XenoMouse™ previously immunized with antigen, were fused with the non secretory NSO myeloma cells transfected with *bcl-2* (NSO-bc12) as described in Galfre G, et *al.,* Methods in Enzymology 73, 3-46, (1981). Briefly, the fusion was performed by mixing washed spleen cells and myeloma cells at a ratio of 5:1 and gently pelleting them by centrifugation at 800Xg. After complete removal of the supernatant the cells were treated with 1 ml of 50% PEG/DMSO (polyethylene glycol MW 1500, 10% DMSO, Sigma) which was added over 1 min., the mixture was further incubated for one minute, and gradually diluted with 2 ml of DMEM over 2 minutes and diluted further with 8 ml of DMEM over 3 minutes. The process was performed at 37 °C with continued gentle stirring. After fusion the cells were resuspended in DMEM, 15% FCS, containing HAT, and supplemented with L glutamine, pen/strep, for culture at 37 °C and 10% CO2 in air. Cells were plated in flat bottomed 96 well microtiter trays. Cultures were maintained in HAT supplemented media for 2 weeks before transfer to HT supplemented media. Cultures were regularly examined for hybrid cell growth, and supernatants from those wells containing hybridomas were collected for a primary screen analysis for the presence of human μ, human gamma 2, and human kappa chains in an antigen specific ELISA as described above. Positive cultures were transferred to 48 well plates and when reaching confluence transferred to 24 well plates. Supernatants were tested in an antigen specific ELISA for the presence of human μ, human gamma 2, and human kappa chains.

As shown in Table 3 several hybridomas secreting fully human monoclonal antibodies with specificity for human IL-8 have been generated from representative fusions. In all of these human monoclonal antibodies the human gamma-2 heavy chain is associated with the human kappa light chain.

TABLE 3: ELISA determination of heavy and light chain composition of anti-IL-8 human monoclonal antibodies generated in XenoMouse™

| Sample ID | Ig class | reactivity to hIL8 | | | | Total hIgG |
|---|---|---|---|---|---|---|
| | | titers | Hκ OD (1:1) | mλ OD (1:1) | hγ OD (1:1) | (ng/ml) |
| Bkgd | | | 0.08 | 0.04 | 0.12 | |
| I8D1.1 | hIgG2 | 500 | 4.12 | 0.04 | 4.09 | 1,159 |
| I8K2.1 | hIgG2 | 200 | 4.18 | 0.18 | 4.11 | 2,000 |
| I8K2.2 | hIgG2 | 1,000 | 4.00 | 0.04 | 4.00 | 4,583 |

(continued)

| Sample ID | Ig class | reactivity to hIL8 | | | | Total hIgG |
|---|---|---|---|---|---|---|
| | | titers | $H_\kappa$ OD (1:1) | mλ OD (1:1) | hγ OD (1:1) | (ng/ml) |
| I8K4.2 | hIgG2 | 200 | 3.98 | 0.04 | 3.49 | 450 |
| I8K4.3 | hIgG2 | 200 | 3.80 | 0.05 | 4.09 | 1,715 |
| I8K4.5 | hIgG2 | 1,000 | 4.00 | 0.06 | 4.00 | 1,468 |

Evaluation of kinetic constants of XenoMouse™ hybridomas

In order to determine the kinetic parameters of these antibodies, specifically their on and off rates and their dissociation constants (KD), they were analyzed on the BIAcore instrument (Pharmacia). The BIAcore instrument uses plasmon resonance to measure the binding of an antibody to an antigen-coated gold chip.

BIAcore reagents and instrumentation:

The BIAcore instrument, CM5 sensor chips, surfactant P20, and the amine coupling kit containing N-hydroxysuccinimide (NHS), N-ethyl-N'-(3-diethylaminopropyl)-carbodimide (EDC), and ethanolamine were purchased from Pharmaicia Biosensor. Immobilization of human recombinant IL-8 onto the sensor surface was carried out at low levels of antigen density immobilized on the surface and was performed according to the general procedures outlined by the manufacturers. Briefly, after washing and equilibrating the instrument with HEPES buffer (HBS; 10 mM HEPES, 150 mM NaCl, 0.05% surfactant P20, pH 7.4) the surface was activated and IL-8 immobilized for the subsequent binding and kinetic studies. The sensor surface was activated with 5 μl of a mixture of equal volumes of NHS (0.1 M) and EDC (0.1 M) injected at 10 μl/min across the surface for activation, then 5 μl of the ligand (human recombinant IL-8) at 12 μg/ml in 5 mM maleate buffer, pH 6.0 was injected across the activated surface, and finally non-conjugated active sites were blocked with an injection of 35 μl of 1 M ethanolamine. The surface was washed to remove non-covalently bound ligand by injection of 5 μl 0.1 M HCl. All the immobilization procedure was carried out with a continuous flow of HBS of 10 μl/min. About 100 resonance units (RU) of ligand (82 and 139 RU, in separate experiments) were immobilized on the sensorship, (according to the manufacturers 1,000 RU corresponds to about 1 $ng/mm^2$ of immobilized protein).

These ligand coated surfaces were used to analyze hybridoma supernatants for their specific binding to ligand and for kinetic studies. The best regenerating condition for the analyte dissociation from the ligand in these sensorships was an injection of 10 μl 100 mM HCl with no significant losses of binding observed after many cycles of binding and regeneration.

Determination of the dissociation, and association rates and the apparent affinity constants of fully human monoclonal antibodies specific for IL-8.

The determination of kinetic measurements using the BIAcore in which one of the reactants is immobilized on the sensor surface was done following procedures suggested by the manufacturers and described in Karlsson et al. "Kinetic analysis of monoclonal antibody-antigen interaction with a new biosensor based analytical system." J. Immunol. Methods (19910 145, 229. Briefly the single site interaction between two molecules A and B is described by the following equation.

$$d[AB]/dt=ka[A][B]-kd[AB]$$

In which B is immobilized on the surface and A is injected at a constant concentration C. The response is a measure of the concentration of the complex [AB] and all concentration terms can be expressed as Response Units (RU) of the BIAcore:

$$dR/dt-kaC(Rmax-R) - kdR$$

where dR/dt is the rate of change of the signal, C is the concentration of the analyte, Rmax is the maximum analyte binding capacity in RU and R is the signal in RU at time t. In this analysis the values of ka and kd are independent of the concentration of immobilized ligand on the surface of the sensor. The dissociation rates (kd) and association rates (ka) were determined using the software provided by the manufacturers, BIA evaluation 2.1. The dissociation rate constant was measured during the dissociation phase that extended for 10 minutes at a constant buffer flow rate of 45 ul/min, after the completion of the injection of the hybridoma supernatants onto the surface containing immobilized IL-8. The association phase extended over 1.25 minutes at a flow rate of 45 ul/min and the data was fitted into the model using the previously determined kd values. At least two surfaces with different levels of immobilized ligand were used in which different concentrations of anti IL-8 hybridoma supernatants were tested for binding and analyzed for kinetic data. The kinetic constants determined on these two surfaces are presented in Table 4. The affinities were determined to be very, ranging from 7 X $10^{-11}$ to 2 X $10^{-9}$ M. This compares vary favorably with the affinities of murine monoclonal antibodies derived from normal mice.

TABLE 4: Kinetic constants of fully human monoclonal antibodies (IgG2, kappa) derived from XenoMouse™ II-a with specificity to human IL-8, determined by BIAcore.

| Hybridoma | association rate ka ($M^{-1}s^{-1}$) | dissociation rate kd ($s^{-1}$) | Dissociation Constant KD (M) =kd/ka | BIAcore surface h-IL-8 [RU] |
|---|---|---|---|---|
| **I8D1-1** | 3.36 x 106<br>2.80 x 106 | 2.58 x 10-4<br>1.73 x 10-4 | 7.70 x 10-11<br>6.20 x 10-11 | 81<br>134 |
| **I8K2-1** | 4.38 x 105<br>3.83 x 105 | 6.73 x 10-4<br>6.85 x 10-4 | 1.54 x 10-9<br>1.79 x 10-9 | 81<br>134 |
| **I8K2-2** | 5.24 x 105<br>4.35 x 105 | 2.26 x 10-4<br>2.30 x 10-4 | 4.30 x 10-10<br>5.30 x 10-10 | 81<br>134 |
| **I8K4-2** | 5.76 x 106<br>1.95 x 106 | 8.17 x 10-4<br>3.84 x 10-4 | 1.42 x 10-10<br>1.96 x 10-10 | 81<br>134 |
| **I8K4-3** | 2.66 x 106<br>1.46 x 106 | 7.53 x 10-4<br>5.72 x 10-4 | 2.83 x 10-10<br>3.90 x 10-10 | 81<br>134 |
| **I8K4-5** | 4.00 x 105<br>1.70 x 105 | 9.04 x 10-4<br>4.55 x 10-4 | 2.26 x 10-9<br>2.68 x 10-9 | 81<br>134 |

Methods for isolation of human neutrophils and assays for antibody activity

The primary in vivo function of IL-8 is to attract and activate neutrophils. Neutrophils express on their surface two distinct receptors for IL-8, designated the A receptor and the B receptor. In order to determine whether the fully human antibodies could neutralize the activity of IL-8, two different in vitro assays were performed with human neutrophils. In one assay, the ability of the antibodies to block binding or radiolabelled IL-8 to neutrophil IL-8 receptors was tested. In a second assay, the antibodies were tested for their ability to block an IL-8-induced neutrophil response, namely the upregulation of the integrin Mac-1 on the neutrophil surface. Mac-1 is composed of two polypeptide chains, CD11b and CD18. Typically, anti-CD11b antibodies are used for its detection.

Isolation of neutrophils:

Human neutrophils are isolated from either freshly drawn blood or buffy coat. Human blood is collected by venipuncture into sterile tubes containing EDTA. Buffy coats are obtained from Stanford Blood Bank. They are prepared by centrifuging anticoagulated blood (up to 400 ml) in plastic bags at 2600 xg for 10 min at 20˚C with the brake off. The plasma supernatant is aspirated out of the bag and the buffy coat, i.e., the upper cell layer (40-50 ml/bag) is collected. One unit of buffy coat (40-50 ml) is diluted to final volume of 120 ml with $Ca^{2+}$, $Mg^{2+}$-free PBS. 30 milliliters of blood or diluted buffy coat are transferred into 50-ml centrifuge tubes on top of a 20-ml layer of Ficoll-Paque Plus (Pharmacia Biotech). The tubes are centrifuged at 500 xg for 20 min at 20˚C with brake off. The supernatant, the mononuclear cells at the interface, and the layer above the pellet are carefully withdrawn. To completely remove the mononuclear cells, the cell pellet containing neutrophils and erythrocytes is resuspended with 5 ml of PBS and transferred into clean 50-ml tubes. The cells are washed in $Ca^{2+}$, $Mg^{2+}$-free PBS (300 xg for 5 min at 4˚C). The erythrocytes are then lysed with ammonium chloride. The cells are resuspended in 40 ml of an ice-cold solution containing 155 mM $NH_4Cl$ and 10 nM EDTA, pH 7.2-7.4. The tubes are kept on ice for 10 min with occasional mixing and then centrifuged at 300 xg for 5 min at 4˚C. The pellet is resuspended in PBS and washed once (300 xg for 5 min at 4˚C). If erythrocyte lysis appears incomplete, the treatment with ammonium chloride is repeated. The neutrophils are again washed and finally suspended either in assay medium (RPMI-1640 supplemented with 10% fetal calf serum, 2 mM L-glutamine, $5x10^{-5}$ 2-mercap-thoethanol, 1X nonessential amino acids, 1 mM sodium pyruvate and 10 mM Hepes) at a density of $3x10^7$ cells/ml or in a binding buffer (PBS containing 0.1% bovine serum albumin and 0.02% $NaN_3$), at a density of $6x10^6$ cells/ml.

IL-8 receptor binding assay:

Multiscreen filter plates (96-well, Millipore, MADV N6550) were pretreated with a PBS binding buffer containing 0.1% bovine serum albumin and 0.02% NaN, at 25˚C for 2 hours. A final volume of 150 µl, containing $4x10^5$ neutrophils, 0.23 nM [$^{125}$I]-human-IL-8 (Amersham, IM-249) and varying concentrations of antibodies made up in PBS binding buffer, was added to each well, and plates were incubated for 90 min at 4˚C. Cells were washed 5 times with 200 µl of ice-cold PBS, which was removed by aspiration. The filters were air-dried, 3.5 ml of scintillation fluid was added (Beckman Ready Safe) and filters were counted on a Beckman LS6000IC counter. The data obtained is presented as % specific bound [$I^{125}$]-IL-8, which is calculated as the cpm in the presence of antibody divided by the cpm in the presence of PBS binding buffer only and multiplied by 100 (Figure 15). All six of the human anti-IL-8 monoclonals tested blocked IL-8 binding to human neutrophils.

Neutrophil CD11b (Mac-1) expression assay:

Human IL-8 at a final concentration of 10 nM was preincubated with varying concentrations of monoclonal antibodies at 4˚C for 30 minutes and at 37˚C for an additional 30 min. Neutrophils ($4x10^5$/well) were exposed to IL-8 in the presence or absence of antibodies at 4˚C for 90 min, and incubated with PE-conjugated mouse-anti-human-CD11b (Becton Dickinson) for 45 min at 4˚C. The cells were washed with ice-cold PBS containing 2% fetal calf serum. Fluorescence was measured on a Becton Dickinson FACscan cell analyzer. A mouse monoclonal antibody against human CD11b obtained from R&D System, Inc. was used as a positive control while the purified myeloma human IgG2 (Calbiochem) was used as a negative control in the experiments. The expression levels of CD11b on neutrophils were measured and expressed as the mean fluorescence channel. The mean fluorescence channel derived form the negative control antibody was subtracted from those of experimental samples.

$$\% \text{ inhibition} = \frac{\text{mean fluorescence in presence of IL-8 only} - \text{mean fluorescence in the presence of antibodies}}{\text{mean fluorescence in the presence of IL-8 only} - \text{mean fluorescence in the presence of human IgG2}} \times 100$$

As shown in Table 5, five of the six antibodies blocked upregulation of CD11b to some degree, with three of the five giving complete blocking.

TABLE 5: Inhibition of CD11b expression on human neutrophils by monoclonal antibodies against IL-8.

| Antibody | Concentration (nM) | Inhibition of CD11b expression (%) |
|---|---|---|
| R&D anti-IL8 | 333 | 100 |
| I8K1.1 | 6 | 100 |
| I8K2.1 | 10 | 60 |
| I8K2.2 | 32 | 100 |
| I8K4.2 | 3 | 10 |
| I8K4.3 | 8 | 100 |
| I8K4.5 | 5 | 0 |
| Human IgG2 | 33 | 0 |

Background of CD11b expression is 670 (mean fluorescence) while CD11b expression in the presence of 10 nM of human IL-8 is 771.

Sequence analysis of Immunoglobulin transcripts derived from anti-hIL-8 hybridomas.

All sequences were derived by direct sequencing of PCR fragments generated form RT-PCR reactions of RNA prepared from hybridomas D1.1, K2.2, K4.2 and K4.3, using human $V_H$ and human V, family specific primers (Marks et. al. 1991; Euro J. Immunol 21;985-991) and a primer specific for either the human gamma 2 constant region (MG-40d; 5'GCTGAGGGAGTAGAGTCCTGAGGACTGT-3') or human kappa constant region (HKP2; Green et al 1994; Nature Genetics 7: 13-21)). In Figure 16 A-H, both strands of the four clones were sequenced and analyzed to generate the complete sequence. All sequences were analyzed by alignments to the "V BASE sequence directory", Tomlinson et al., MRC Centre for Protein Engineering, Cambridge, UK. The variable and joining regions are indicated by brackets []. Nucleotides containing an "N" indicate uncertainty in the generated sequence.

Based on sequence alignments with sequences found in the V-base database the heavy chain transcript from hybridoma D1.1 has a human $V_H$4-21(DP-63) variable region (7 point mutations were observed compared to the germline sequence), a human 21-10rc D segment, a human $J_H$3 joining region and a human gamma 2 constant region. See Figure 16A.

The kappa light chain transcript from hybridoma D1.1 is comprised of a human kappa variable region with homology to $V_k$ 08/018 (DPK1) (16 point mutations were observed when compared to the germline sequence) a human $J_k$3 joining region, and a human kappa constant region. See Figure 16B.

Based on sequence alignments with sequences found in the V-base database the heavy chain transcript from hybridoma K2.2 has a human $V_H$3-30 variable region (3 point mutations were observed compared to the germline sequence), a human IR3rc D segment, a human $J_H$4 joining region and a human gamma 2 constant region. See Figure 16C.

The kappa light chain transcript from hybridoma K2.2 is comprised of a human kappa variable region with homology to $V_k$IV (B3; DPK24) (9 point mutations were observed when compared to the germline sequence), a human $J_K$3 joining region, and a human kappa constant region. See Figure 16D.

Based on sequence alignments with sequences found in the V-base database the heavy chain transcript from hybridoma K4.2 has a human $V_H$4-34 variable region (8 point mutations were observed compared to the germline sequence), a human K1 D segment, a human $J_H$4 joining region and a human gamma 2 constant region. See Figure 16E.

The kappa light chain transcript from hybridoma K4.2 is comprised of a human kappa variable region with homology to V, 08/018 (DPK1) (6 point mutations were observed when compared to the germline sequence), a human $J_k$4 joining region, and a human kappa constant region. See Figure 16F.

Based on sequence alignments with sequences found in the V-base database the heavy chain transcript from hybridoma K4.3 has a human $V_H$5-51 (DP-73) variable region, a human M5-a/M5-b D segment, a human $J_H$4 joining region and a human gamma 2 constant region. See Figure 16G.

The kappa light chain transcript from hybridoma K4.3 is comprised of a human kappa variable region with homology to V, 02/012 (DPK9) (9 point mutations were observed when compared to the germline sequence), a human $J_k$4 joining region, and a human kappa constant region. See Figure 16H.

Biological Deposits

yH1C contained in *S. cerivisiae* was deposited with the American Type Culture Collection ("ATCC"), 12301 Parklawn Drive, Rockville MD 20852, USA, on April 26, 1996, and given ATCC accession no. 74367. The deposit of this YAC is for exemplary purposes only, and should not be taken as an admission by the Applicant that such deposit is necessary for enablement of the claimed subject matter.

In respect of all designated States in which such action is possible and to the extent that it is legally permissible under the law of the designated State, it is requested that a sample of the deposited micro-organism be made available only by the issue thereof to an independent expert, in accordance with the relevant patent legislation, e.g., EPC rule 28(4).

SEQUENCE LISTING

<110> Abgenix, Inc.

<120> HUMAN ANTIBODIES DERIVED FROM IMMUNIZED XENOMICE

<130> EP37734HVpau

<140> EP 05 028 220.1
<141> 2005-12-21

<150> EP 96 913 247.1
<151> 1996-04-29

<150> PCT/US96/05928
<151> 1996-04-29

<150> 08/430,938
<151> 1995-04-27

<160> 21

<170> PatentIn Ver. 3.3

<210> 1
<211> 259
<212> DNA
<213> Homo sapiens

<400> 1

```
agaccctctc actcacctgt gccatctccg gggacagtgt ctctagcaac agtgctgctt 60
ggaactggat caggcagtcc ccatcgagag gccttgagtg gctgggaagg acatactaca 120
ggtccaagtg gtataatgat tatgcagtat ctgtgaaaag tcgaataacc atcaacccag 180
acacatccaa gaaccagttc tccctgcagc tgaactctgt gactcccgag gacacggctg 240
tgtattactg tgcaagaga                                             259
```

<210> 2
<211> 400
<212> DNA
<213> Homo sapiens

<400> 2

```
agaccctctc actcacctgt gccatctccg gggacagtgt ctctagcgac agtgctgctt 60
ggaactggat caggcagtcc ccatcgagag gccttgagtg gctgggaagg acatactaca 120
ggtccaagtg gtataatgat tatgcagttt ctgtgaaaag tcgaataacc atcaacccag 180
acacatccaa gaaccagttc tccctgcagc tgaactctgt gactcccgag gacacggctg 240
tgtattactg tgcaagagat atagcagtgg ctggcgtcct ctttgactgc tggggccagg 300
gaaccctggt caccgtctcc tcagggagtg catccgcccc aacccttttc cccctcgtct 360
cctgtgagaa ttccccgtcg gatacgagca gcgtggccgt                       400
```

<210> 3
<211> 43
<212> DNA
<213> Homo sapiens

<400> 3
cttgactagc tggggccaag gaaccctggt caccgtctcc tca 43

<210> 4
<211> 15

<212> DNA
<213> Homo sapiens

<400> 4
tatagcagca gctgg 15

<210> 5
<211> 77
<212> DNA
<213> Homo sapiens

<400> 5

gggagtgcat ccgccccaac ccttttcccc ctcgtctcct gtgagaattc cccgtcggat 60
acgagcagcg tggccgt 77

<210> 6
<211> 302
<212> DNA
<213> Homo sapiens

<400> 6

gacatcgtga tgacccagtc tccagactcc ctggctgtgt ctctgggcga gagggccacc 60
atcaactgca agtccagcca gagtgtttta tacagctcca acaataagaa ctacttagct 120
tggtaccagc agaaaccagg acagcctcct aagctgctca tttactgggc atctacccgg 180
gaatccgggg tccctgaccg attcagtggc agcgggtctg ggacagattt cactctcacc 240
atcagcagcc tgcaggctga agatgtggca gtttattact gtcagcaata ttatagtact 300
cc 302

<210> 7
<211> 442
<212> DNA
<213> Homo sapiens

<400> 7

accatcaagt gcaagtccag ccagagtgtt ttgtacactt ccagcaataa gaactactta 60
gcttggtacc agcagaaacc aggacagcct cctaaactac tcatttactg ggcatctacc 120
cgggaatccg gggtccctga ccgattcagt ggcagcgggt ctgggacaga tttcactctc 180
accatccgca gcctgcaggc tgaagatgtg gcagtttatt actgtcagca atattatact 240
attccattca atttcggccc tgggaccaga gtggatatca aacgaactgt ggctgcacca 300
tctgtcttca tcttcccgcc atctgatgag cagttgaaat ctggaactgc ctctgttgtg 360
tgcctgctga ataacttcta tcccagagag gccaaagtac agtggaaggt ggataacgcc 420
ctccaatcgg gttggggaaa aa 442

<210> 8
<211> 38
<212> DNA
<213> Homo sapiens

<400> 8
attcactttc ggccctggga ccaaagtgga tatcaaac 38

<210> 9
<211> 149

<212> DNA
<213> Homo sapiens

<400> 9

```
gaactgtggc tgcaccatct gtcttcatct tcccgccatc tgatgagcag ttgaaatctg 60
gaactgcctc tgttgtgtgc ctgctgaata acttctatcc cagagaggcc aaagtacagt 120
ggaaggtgga taacgccctc caatcgggt                                   149
```

<210> 10
<211> 400
<212> DNA
<213> Homo sapiens

<400> 10

```
cctgtccctc acctgcgctg tctatggtgg gtccttcagt ggttactact ggagctggat 60
ccgccagccc ccagggaagg gactggagtg gattggggaa atcaatcaaa gtggaaggca 120

ccaattacaa cccgtccctc aagagtcgag tcatcatatc aatagacacg tccaagaccc 180
agttctccct gaagttgagc tctgtgaccg ccgcggacac ggctgtgtat tactgtgcga 240
gagagactcc ccatgctttt gatatctggg gccaagggac aatggtcacc gtctcttcag 300
cctccaccaa gggcccatcg gtcttccccc tggcgccctg ctccaggagc acctccgaga 360
gcacagcgcg ccctgggctg cctggtcaag gactacttcc                       400
```

<210> 11
<211> 444
<212> DNA
<213> Homo sapiens

<220>
<221> modified_base
<222> (255)
<223> a, c, g, t, unknown or other

<400> 11

```
cagtctccat cctccctgtc tgcatctgta ggcgacagag tcaccatcac ttgccaggcg 60
agtcaggaca ttagtaagtt tttaagttgg tttcaacaga aaccagggaa agcccctaaa 120
ctcctgatct acggtacatc ctatttggaa accggggtcc catcaagttt cagtggaagt 180
ggatctggga cagattttac tctcaccatc agcagcctgc agcctgaaga tgttgcaaca 240
tatttctgta acagnatgat gatctcccat acactttcgg ccctgggacc aaagtggata 300
tcaaacgaac tgtggctgca ccatctgtct tcatcttccc gccatctgat gagcagttga 360
aatctggaac tgcctctgtt gtgtgcctgc tgaataactt ctatcccaga gaggccaaag 420
tacagtggaa ggtggataac gccc                                        444
```

<210> 12
<211> 453
<212> DNA
<213> Homo sapiens

<220>

<221> modified_base
<222> (64)
<223> a, c, g, t, unknown or other

<400> 12

```
aggtccctga gactctcctg tgcagcctct ggattcacct tcagtagcta tggcatgcac 60
tggntccgcc aggctccagg caaggggctg gagtgggtgg cagaaatatc atatgatgga 120
agtaataaat actatgtaga ctccgtgaag ggccgactca ccatctccag agacaattcc 180
aagaacacgc tgtatctgca aatgaacagc ctgagagctg aggacacggc tgtgtattac 240
tgtgcgagag accgactggg gatctttgac tactggggcc agggaaccct ggtcaccgtc 300
tcctcagcct ccaccaaggg cccatcggtc ttccccctgg cgccctgctc caggagcacc 360
tccgagagca cagcgcggcc ctgggctgcc tggtccaagg actacttccc ccgaaccggt 420
gacggtgtcg tggaactcag gcgctctgac cag                              453
```

<210> 13
<211> 470
<212> DNA
<213> Homo sapiens

<220>
<221> modified_base
<222> (6)
<223> a, c, g, t, unknown or other

<220>
<221> modified_base
<222> (460)
<223> a, c, g, t, unknown or other

<400> 13

```
ctgacncagt ctccagactc cctggctgtg tctctgggcg agagggccac catcaactgc 60
aagtccagcc agagtgtttt atacatctcc aacaataaaa ctacttagct tggtaccagc 120
agaaaccagg acagtctcct aaactgctca tttactgggc atctacccgg aaatccgggg 180
tccctgaccg attcagtggc agcgggtctg ggacagattt cactctcacc atcagcagcc 240

tgcaggctga agatgtggca gtttattact gtcaacagta ttatgatact ccattcactt 300
tcggccctgg gaccaaagtg gatatcaaac gaactgtggc tgcaccatct gtcttcatct 360
tcccgccatc tgatgagcag ttgaaatctg gaactgcctc tgttgtgtgc ctgctgaata 420
acttctatcc cagagaggcc aaagtacagt ggaaggtggn taacgcccca            470
```

<210> 14
<211> 462
<212> DNA
<213> Homo sapiens

<400> 14

```
tccctcacct gcgctgtcta tggtgggtcc ttcagtggtt actactggac ctggatccgc 60
cagcccccag ggaaggggct ggagtggatt ggggaaatca ttcatcatgg aaacaccaac 120
tacaacccgt ccctcaagag tcgagtctcc atatcagttg acacgtccaa gaaccagttc 180
tccctgacac tgagctctgt gaccgccgcg gacacggctg tgtattactg tgcgagaggg 240
ggagcagtgg ctgcgtttga ctactggggc cagggaaccc tggtcaccgt ctcctcagcc 300
tccaccaagg gcccatcggt cttccccctg gcgccctgct ccaggagcac ctccgagagc 360
acagcgcggc cctgggctgc ctggtcaagg actacttccc ccgaaccggt gacggtgtcg 420
tggaactcag gcgctctgac cagcggcgtg cacaccttcc ca                   462
```

<210> 15
<211> 437
<212> DNA
<213> Homo sapiens

<400> 15

```
tgacccagtc tccatcctcc ctgtctgcat ctgtaggaga cagagtcacc atcacttgcc 60
aggcgagtca ggacattagt aactatttaa attggtatca acagaaagca gggaaagccc 120
ctaaggtcct gatctacgct gcatccaatt tggaagcagg ggtcccatca aggttcagtg 180
gaagtggatc tgggacagat tttactttca ccatcagcag cctgcagcct gaagatattg 240
caacatatta ttgtcaacac tatgataatc tactcacttt cggcggaggg accaaggtag 300
agatcaaacg aactgtggct gcaccatctg tcttcatctt cccgccatct gatgagcagt 360
tgaaatctgg actgcctctg ttgtgtgcct gctgaataac ttctatccca gagaggccaa 420
agtacagtgg aaggtgg                                                437
```

<210> 16
<211> 477
<212> DNA
<213> Homo sapiens

<400> 16

```
agtctctgaa gatctcctgt aagggttctg gatacagctt taccagctac tggatcggct 60
gggtgcgcca gatgcccggg aaaggcctgg agtggatggg gatcatctat cctggtgact 120
ctgataccag atacagcccg tccttccaag gccaggtcac catctcagcc gacaagtcca 180
tcagcaccgc ctacctgcag tggagcagcc tgaaggcctc ggacaccgcc atgtattact 240
gtgcgagaca ggacggtgac tcctttgact actggggcca gggaaccctg gtcaccgtct 300
cctcagcctc caccaagggc ccatcggtct tccccctggc gccctgctcc aggagcacct 360
ccgagagcac agcgcggccc tgggctgcct ggtccaagga ctacttcccc cgaaccggtg 420
acggtgtcgt ggaactcagg cgctctgacc agcggcgtgc acaccttccc actgcca    477
```

<210> 17
<211> 410
<212> DNA
<213> Homo sapiens

<220>
<221> modified_base
<222> (151)
<223> a, c, g, t, unknown or other

<220>
<221> modified_base
<222> (212)
<223> a, c, g, t, unknown or other

<220>
<221> modified_base

<222> (270)
<223> a, c, g, t, unknown or other

<220>
<221> modified_base
<222> (279)
<223> a, c, g, t, unknown or other

<400> 17

```
tgtctgcatc tattggagac agagtcacca tcacttgccg ggcaagtcag agcattagca 60
actatttaaa ttggtatcag cagaaaccag ggcaaagccc ctaagttcct gatctatggt 120
gcatccagtt tggaaagtgg ggtcccatca nggttcagtg gcagtggatc tgggacagat 180
ttcactctca ccatcagcag cctgcaacct gnggattttg caacttacta ctgtcaacag 240
agttacagta accctctcac tttcggcggn gggaccaang tggagatcaa acgaactgtg 300
gctgcaccat ctgtcttcat cttcccgcca tctgatgagc agttgaaatc tggaactgcc 360
tctgttgtgt gcctgctgaa taacttctat cccagagagg ccaaagtaca             410
```

<210> 18
<211> 24
<212> DNA
<213> Artificial Sequence

<220>
<223> Description of Artificial Sequence: synthetic primer

<400> 18
ctctgtgaca ctctcctggg agtt 24

<210> 19
<211> 26
<212> DNA
<213> Artificial sequence

<220>
<223> Description of Artificial sequence: synthetic primer

<400> 19
gaaacgacac tcacgcagtc tccagc 26

<210> 20
<211> 24
<212> DNA
<213> Artificial Sequence

<220>
<223> Description of Artificial Sequence: synthetic primer

<400> 20
ttttctttgt tgccgttggg gtgc 24

<210> 21
<211> 28
<212> DNA
<213> Artificial Sequence

<220>

<223> Description of Artificial Sequence: synthetic primer

<400> 21
gctgagggag tagagtcctg aggactgt 28

# American Type Culture Collection

12301 Parklawn Drive • Rockville, MD 20852 USA • Telephone: (301)231-5520 Telex: 898-055 ATCCNORTH • FAX: 301-770-2587

BUDAPEST TREATY ON THE INTERNATIONAL RECOGNITION OF THE DEPOSIT OF MICROORGANISMS FOR THE PURPOSES OF PATENT PROCEDURE

*INTERNATIONAL FORM*

RECEIPT IN THE CASE OF AN ORIGINAL DEPOSIT ISSUED PURSUANT TO RULE 7.3 AND VIABILITY STATEMENT ISSUED PURSUANT TO RULE 10.2

**To: (Name and Address of Depositor or Attorney)**

Cell Genesys, Inc.
Attn: Michael Mendez
344 Lakeside Drive
Foster City, CA 94404

**Deposited on Behalf of:** Cell Genesys, Inc.

| **Identification Reference by Depositor:** | **ATCC Designation** |
|---|---|
| *Saccharomyces cerevisiae* phase IclgH | 74367 |

The deposit was accompanied by: ___ a scientific description X a proposed taxonomic description indicated above.

The deposit was received April 26, 1996 by this International Depository Authority and has been accepted.

**AT YOUR REQUEST:**

X We will inform you of requests for the strain for 30 years.

The strain will be made available if a patent office signatory to the Budapest Treaty certifies one's right to receive, or if a U.S. Patent is issued citing the strain, and ATCC is instructed by the United States Patent & Trademark Office or the depositor to release said strain.

If the culture should die or be destroyed during the effective term of the deposit, it shall be your responsibility to replace it with living culture of the same.

The strain will be maintained for a period of at least 30 years from date of deposit, or five years after the most recent request for a sample, whichever is longer. The United States and many other countries are signatory to the Budapest Treaty.

The viability of the culture cited above was tested May 8, 1996. On that date, the culture was viable.

**International Depository Authority:** American Type Culture Collection, Rockville, Md. 20852 USA

**Signature of person having authority to represent ATCC:**

Barbara M. Hailey

Barbara M. Hailey, Administrator, Patent Depository

Date: May 9, 1996

cc: Karen Krupen
Geoff Davis

## Claims

1. An antibody or antigen binding portion thereof that specifically binds IL-8 selected from the group consisting of:

(a) an antibody or antigen binding portion thereof, wherein the heavy chain comprises the amino acid sequence encoded by nucleotides of the variable region, the D segment and the joining region set forth in Fig. 16 A, and wherein the light chain comprises the amino acid sequence encoded by nucleotides of the variable region and the joining region set forth in Fig. 16 B;
(b) an antibody or antigen binding portion thereof, wherein the heavy chain comprises the amino acid sequence encoded by nucleotides of the variable region, the D segment and the joining region set forth in Fig. 16 C, and wherein the light chain comprise the amino acid sequence encoded by nucleotides of the variable region and the joining region set forth in Fig. 16 D;
(c) an antibody or antigen binding portion thereof, wherein the heavy chain comprises the amino acid sequence encoded by nucleotides of the variable region, the D segment and the joining region set forth in Fig. 16 E, and wherein the light chain comprises the amino acid sequence encoded by nucleotides of the variable region and the joining region set forth in Fig. 16 F; and
(d) an antibody or antigen binding portion thereof, wherein the heavy chain comprises the amino acid sequence encoded by nucleotides of the variable region, the D segment and the joining region set forth in Fig. 16 G, and wherein the light chain comprises the amino acid sequence encoded by nucleotides of the variable region and the joining region set forth in Fig. 16 H.

2. An antibody or antigen binding portion thereof that specifically binds IL-8 selected from the group consisting of:

(a) an antibody or antigen binding portion thereof, wherein the heavy chain comprises the amino acid sequence encoded by the nucleic acid sequence set forth in Fig. 16 A, and wherein the light chain comprises the amino acid sequence encoded by the nucleic acid sequence set forth in Fig. 16 B;
(b) an antibody or antigen binding portion thereof, wherein the heavy chain comprises the amino acid sequence encoded by the nucleic acid sequence set forth in Fig. 16 C, and wherein the light chain comprises the amino acid sequence encoded by the nucleic acid sequence set forth in Fig. 16 D;
(c) an antibody or antigen binding portion thereof, wherein the heavy chain comprises the amino acid sequence encoded by the nucleic acid sequence set forth in Fig. 16 E, and wherein the light chain comprises the amino acid sequence encoded by the nucleic acid sequence set forth in Fig. 16 F; and
(d) an antibody or antigen binding portion thereof, wherein the heavy chain comprises the amino acid sequence encoded by the nucleic acid sequence set forth in Fig. 16 G, and wherein the light chain comprises the amino acid sequence encoded by the nucleic acid sequence set forth in Fig. 16 H.

3. The antibody or antigen binding portion thereof of claim 1 or 2, wherein the antibody or antigen-binding portion thereof reduces IL-8 induced MAC-1 (CD11b) expression on neutrophils.

4. A cell producing the antibody or antigen-binding portion thereof of any one of claims 1 to 3.

5. The cell of claim 4, wherein said cell is derived from a non-human mammal immunized with IL-8, wherein said mammal is capable of producing a fully human antibody to an antigen of interest.

6. The cell of claim 4 or 5, wherein the cell expresses cDNAs encoding said antibody or antigen-binding portion thereof.

7. A method of producing the antibody or antigen binding portion thereof of any one of claims 1 to 3, comprising the step of culturing the cell of any one of claims 4 to 6 and recovering said antibody or antigen-binding portion thereof.

8. A nucleic acid molecule encoding the antibody or antigen binding portion thereof of claims 1-3.

9. A host cell comprising the nucleic acid molecule of claim 8.

10. Pharmaceutical composition comprising the antibody or antigen-binding portion thereof of any one of claims 1 to 3.

11. Use of the antibody or antigen-binding portion thereof of any one of claims 1 to 3 for the preparation of a medicament for reducing binding of IL-8 to neutrophils.

12. The use according to claim 11, wherein IL-8 is to be contacted with the antibody or antigen binding portion thereof of any one of claims 1 to 3.

## Patentansprüche

1. Antikörper oder Antigen-bindender Bereich davon, welcher spezifisch IL-8 bindet, ausgewählt aus der Gruppe bestehend aus:

(a) einem Antikörper oder Antigen-bindenden Bereich davon, wobei die schwere Kette die Aminosäuresequenz umfasst, welche durch Nucleotide der variablen Region, des D-Segments und der Binderegion wie in Fig. 16 A gezeigt codiert wird, und wobei die leichte Kette die Aminosäuresequenz umfasst, welche durch Nucleotide der variablen Region und der Binderegion wie in Fig. 16 B gezeigt codiert wird;
(b) einem Antikörper oder Antigen-bindenden Bereich davon, wobei die schwere Kette die Aminosäuresequenz umfasst, welche durch Nucleotide der variablen Region, des D-Segments und der Binderegion wie in Fig. 16 C gezeigt codiert wird, und wobei die leichte Kette die Aminosäuresequenz umfasst, welche durch Nucleotide der variablen Region und der Binderegion wie in Fig. 16 D gezeigt codiert wird;
(c) einem Antikörper oder Antigen-bindenden Bereich davon, wobei die schwere Kette die Aminosäuresequenz umfasst, welche durch Nucleotide der variablen Region, des D-Segments und der Binderegion wie in Fig. 16 E gezeigt codiert wird, und wobei die leichte Kette die Aminosäuresequenz umfasst, welche durch Nucleotide der variablen Region und der Binderegion wie in Fig. 16 F gezeigt codiert wird; und
(d) einem Antikörper oder Antigen-bindenden Bereich davon, wobei die schwere Kette die Aminosäuresequenz umfasst, welche durch Nucleotide der variablen Region, des D-Segments und der Binderegion wie in Fig. 16 G gezeigt codiert wird, und wobei die leichte Kette die Aminosäuresequenz umfasst, welche durch Nucleotide der variablen Region und der Binderegion wie in Fig. 16 H gezeigt codiert wird.

2. Antikörper oder Antigen-bindender Bereich davon, welcher spezifisch IL-8 bindet, ausgewählt aus der Gruppe bestehend aus:

(a) einem Antikörper oder Antigen-bindenden Bereich davon, wobei die schwere Kette die Aminosäuresequenz umfasst, welche durch die Nucleinsäuresequenz wie in Fig. 16 A gezeigt codiert wird, und wobei die leichte Kette die Aminosäuresequenz umfasst, welche durch die Nucleinsäuresequenz wie in Fig. 16 B gezeigt codiert wird;
(b) einem Antikörper oder Antigen-bindenden Bereich davon, wobei die schwere Kette die Aminosäuresequenz umfasst, welche durch die Nucleinsäuresequenz wie in Fig. 16 C gezeigt codiert wird, und wobei die leichte Kette die Aminosäuresequenz umfasst, welche durch die Nucleinsäuresequenz wie in Fig. 16 D gezeigt codiert wird;
(c) einem Antikörper oder Antigen-bindenden Bereich davon, wobei die schwere Kette die Aminosäuresequenz umfasst, welche durch die Nucleinsäuresequenz wie in Fig. 16 E gezeigt codiert wird, und wobei die leichte Kette die Aminosäuresequenz umfasst, welche durch die Nucleinsäuresequenz wie in Fig. 16 F gezeigt codiert wird; und
(d) einem Antikörper oder Antigen-bindenden Bereich davon, wobei die schwere Kette die Aminosäuresequenz umfasst, welche durch die Nucleinsäuresequenz wie in Fig. 16 G gezeigt codiert wird, und wobei die leichte Kette die Aminosäuresequenz umfasst, welche durch die Nucleinsäuresequenz wie in Fig. 16 H gezeigt codiert wird.

3. Antikörper oder Antigen-bindender Bereich davon nach Anspruch 1 oder 2, wobei der Antikörper oder Antigen-bindende Bereich davon IL-induzierte MAC-1 (CD11b)-Expression auf Neutrophilen reduziert.

4. Zelle, welche den Antikörper oder Antigen-bindenden Bereich davon nach einem der Ansprüche 1 bis 3 produziert.

5. Zelle nach Anspruch 4, wobei die Zelle abgeleitet ist aus einem nicht-menschlichen Säuger, welcher mit IL-8 immunisiert wurde, wobei der Säuger in der Lage ist, einen vollständigen menschlichen Antikörper gegen ein Antigen von Interesse zu bilden.

6. Zelle nach einem der Ansprüche 4 oder 5, wobei die Zelle cDNAs exprimiert, welche den Antikörper oder Antigen-bindenden Bereich davon codieren.

7. Verfahren zur Herstellung des Antikörpers oder Antigen-bindenden Bereichs davon nach einem der Ansprüche 1 bis 3, umfassend den Schritt der Züchtung der Zelle nach einem der Ansprüche 4 bis 6 und der Gewinnung des Antikörpers oder Antigen-bindenden Bereichs davon.

8. Nucleinsäuremolekül, welches den Antikörper oder Antigen-bindenden Bereich davon nach den Ansprüchen 1 bis 3 codiert.

9. Wirtszelle, umfassend das Nucleinsäuremolekül nach Anspruch 8.

10. Arzneimittel, umfassend den Antikörper oder Antigen-bindenden Bereich davon nach einem der Ansprüche 1 bis 3.

11. Verwendung des Antikörpers oder Antigen-bindenden Bereichs davon nach einem der Ansprüche 1 bis 3 für die Herstellung eines Medikaments zur Reduzierung der Bindung von IL-8 an Neutrophile.

12. Verwendung nach Anspruch 11, wobei IL-8 mit dem Antikörper oder Antigen-bindenden Bereich davon nach einem der Ansprüche 1 bis 3 in Kontakt gebracht werden soll.

**Revendications**

1. Anticorps ou partie de liaison à un antigène de celui-ci qui se lie spécifiquement à IL-8 choisi dans le groupe consistant en :

   (a) un anticorps ou une partie de liaison à un antigène de celui-ci, dans lequel la chaîne lourde comprend la séquence d'acides aminés codée par des nucléotides de la région variable, le segment D et la région de jonction indiquée sur la figure 16A, et dans lequel la chaîne légère comprend la séquence d'acides aminés codée par des nucléotides de la région variable et la région de jonction indiquée sur la figure 16B ;
   (b) un anticorps ou une partie de liaison à un antigène de celui-ci, dans lequel la chaîne lourde comprend la séquence d'acides aminés codée par des nucléotides de la région variable, le segment D et la région de jonction indiquée sur la figure 16C, et dans lequel la chaîne légère comprend la séquence d'acides aminés codée par des nucléotides de la région variable et la région de jonction indiquée sur la figure 16D ;
   (c) un anticorps ou une partie de liaison à un antigène de celui-ci, dans lequel la chaîne lourde comprend la séquence d'acides aminés codée par des nucléotides de la région variable, le segment D et la région de jonction indiquée sur la figure 16E, et dans lequel la chaîne légère comprend la séquence d'acides aminés codée par des nucléotides de la région variable et la région de jonction indiquée sur la figure 16F ; et
   (d) un anticorps ou une partie de liaison à un antigène de celui-ci, dans lequel la chaîne lourde comprend la séquence d'acides aminés codée par des nucléotides de la région variable, le segment D et la région de jonction indiquée sur la figure 16G, et dans lequel la chaîne légère comprend la séquence d'acides aminés codée par des nucléotides de la région variable et la région de jonction indiquée sur la figure 16H.

2. Anticorps ou partie de liaison à un antigène de celui-ci qui se lie spécifiquement à IL-8 choisi dans le groupe consistant en :

   (a) un anticorps ou une partie de liaison à un antigène de celui-ci, dans lequel la chaîne lourde comprend la séquence d'acides aminés codée par la séquence d'acides nucléiques indiquée sur la figure 16A, et dans lequel la chaîne légère comprend la séquence d'acides aminés codée par la séquence d'acides nucléiques indiquée sur la figure 16B ;
   (b) un anticorps ou une partie de liaison à un antigène de celui-ci, dans lequel la chaîne lourde comprend la séquence d'acides aminés codée par la séquence d'acides nucléiques indiquée sur la figure 16C, et dans lequel la chaîne légère comprend la séquence d'acides aminés codée par la séquence d'acides nucléiques indiquée sur la figure 16D ;
   (c) un anticorps ou une partie de liaison à un antigène de celui-ci, dans lequel la chaîne lourde comprend la séquence d'acides aminés codée par la séquence d'acides nucléiques indiquée sur la figure 16E, et dans lequel la chaîne légère comprend la séquence d'acides aminés codée par la séquence d'acides nucléiques indiquée sur la figure 16F ; et
   (d) un anticorps ou une partie de liaison à un antigène de celui-ci, dans lequel la chaîne lourde comprend la séquence d'acides aminés codée par la séquence d'acides nucléiques indiquée sur la figure 16G, et dans lequel la chaîne légère comprend la séquence d'acides aminés codée par la séquence d'acides nucléiques indiquée sur la figure 16H.

3. Anticorps ou partie de liaison à un antigène de celui-ci selon la revendication 1 ou 2, dans lequel l'anticorps ou la partie de liaison à un antigène de celui-ci réduit l'expression de MAC-1 induite par IL-8 (CD11b) sur des neutrophiles.

4. Cellule produisant l'anticorps ou la partie de liaison à un antigène de celui-ci selon l'une quelconque des revendications 1 à 3.

5. Cellule selon la revendication 4, dans laquelle ladite cellule est dérivée d'un mammifère non humain immunisé avec IL-8, dans lequel ledit mammifère est capable de produire un anticorps totalement humain à un antigène d'intérêt.

6. Cellule selon la revendication 4 ou 5, dans laquelle la cellule exprime des ADNc codant ledit anticorps ou ladite partie de liaison à un antigène de celui-ci.

7. Procédé de production de l'anticorps ou de la partie de liaison à un antigène de celui-ci selon l'une quelconque des revendications 1 à 3, comprenant l'étape consistant à mettre en culture la cellule selon l'une quelconque des revendications 4 à 6 et à récupérer ledit anticorps ou ladite partie de liaison à un antigène de celui-ci.

8. Molécule d'acide nucléique codant l'anticorps ou la partie de liaison à un antigène de celui-ci selon les revendications 1 à 3.

9. Cellule hôte comprenant la molécule d'acide nucléique de la revendication 8.

10. Composition pharmaceutique comprenant l'anticorps ou la partie de liaison à un antigène de celui-ci selon l'une quelconque des revendications 1 à 3.

11. Utilisation de l'anticorps ou de la partie de liaison à un antigène de celui-ci selon l'une quelconque des revendications 1 à 3, pour la préparation d'un médicament destiné à réduire la liaison de IL-8 à des neutrophiles.

12. Utilisation selon la revendication 11, dans laquelle IL-8 doit être mis en contact avec l'anticorps ou la partie de liaison à un antigène de celui-ci selon l'une quelconque des revendications 1 à 3.

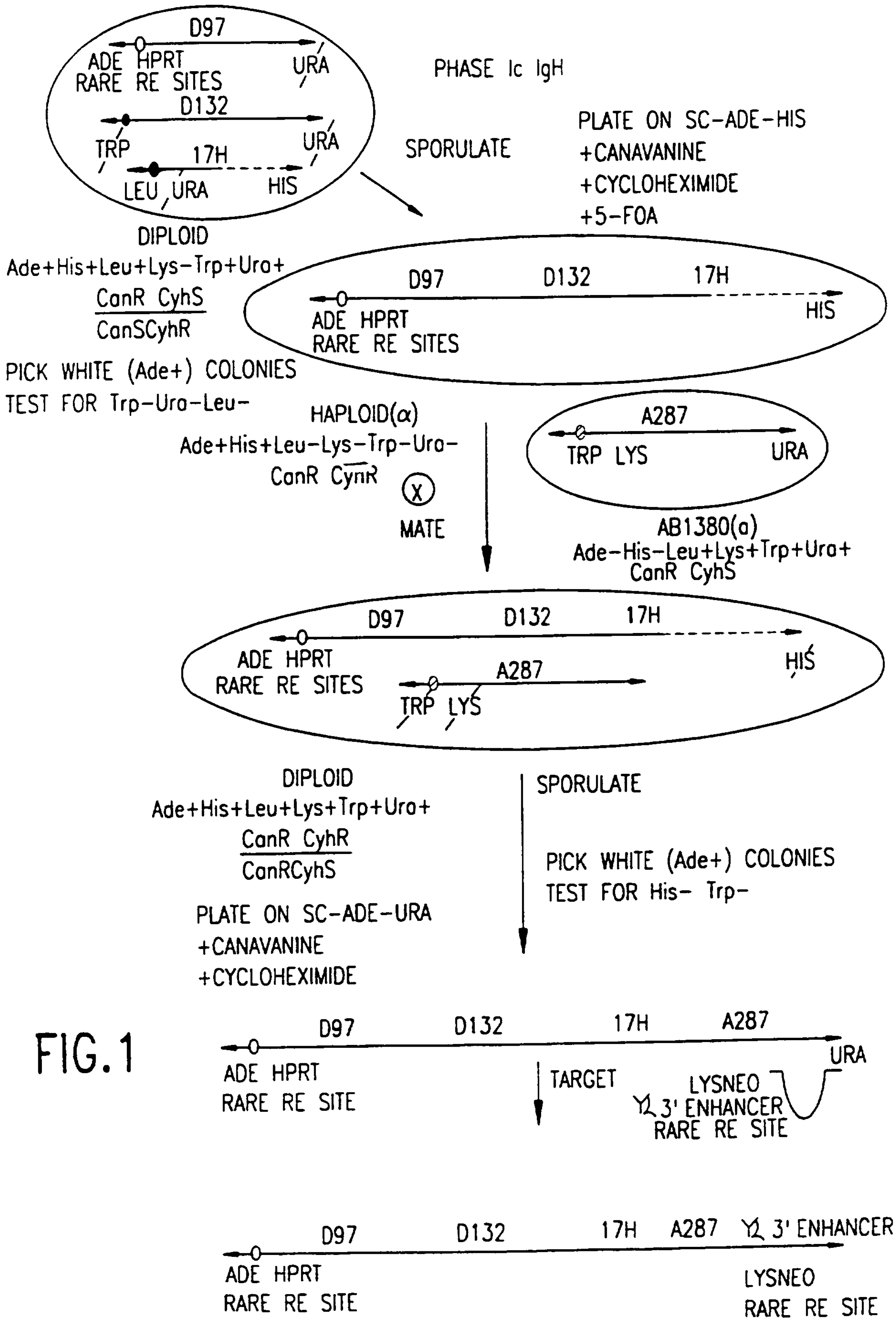
D97
ADE HPRT
RARE RE SITES
URA
D132
TRP
URA
17H
LEU URA
HIS
PHASE Ic IgH
SPORULATE
PLATE ON SC-ADE-HIS
+CANAVANINE
+CYCLOHEXIMIDE
+5-FOA
DIPLOID
Ade+His+Leu+Lys-Trp+Ura+
CanR CyhS
CanSCyhR
D97
D132
17H
ADE HPRT
RARE RE SITES
HIS
PICK WHITE (Ade+) COLONIES
TEST FOR Trp-Ura-Leu-
HAPLOID(α)
Ade+His+Leu-Lys-Trp-Ura-
CanR CynR
X
MATE
A287
TRP LYS
URA
AB1380(a)
Ade-His-Leu+Lys+Trp+Ura+
CanR CyhS
D97
D132
17H
ADE HPRT
RARE RE SITES
HIS
A287
TRP LYS
DIPLOID
Ade+His+Leu+Lys+Trp+Ura+
CanR CyhR
CanRCyhS
SPORULATE
PICK WHITE (Ade+) COLONIES
TEST FOR His- Trp-
PLATE ON SC-ADE-URA
+CANAVANINE
+CYCLOHEXIMIDE
D97
D132
17H
A287
URA
ADE HPRT
RARE RE SITE
TARGET
LYSNEO
Y2 3' ENHANCER
RARE RE SITE
D97
D132
17H
A287
Y2 3' ENHANCER
ADE HPRT
RARE RE SITE
LYSNEO
RARE RE SITE

FIG.1

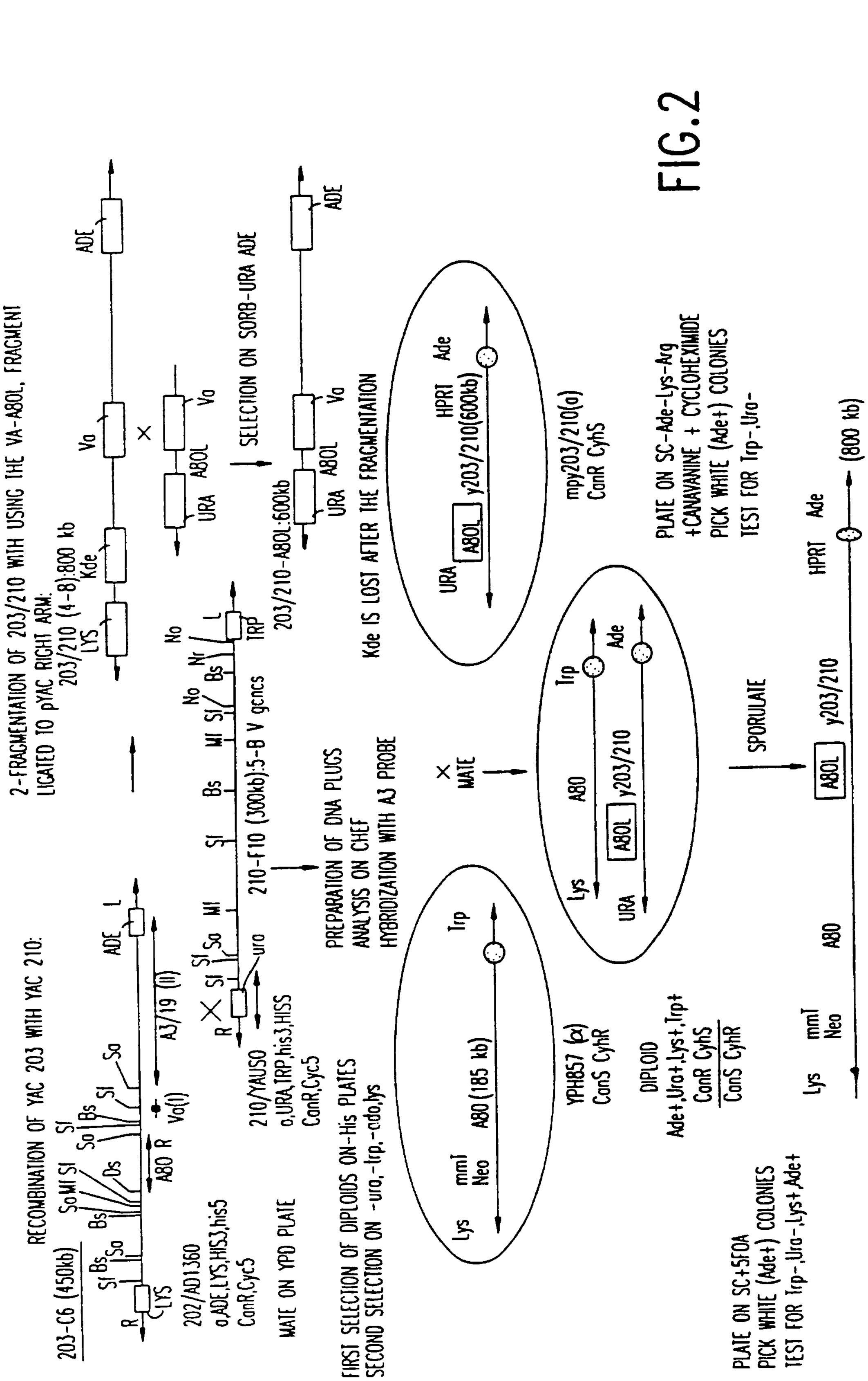
RECOMBINATION OF YAC 203 WITH YAC 210:
203-C6 (450kb)
202/AD1360
o,ADE,LYS,HIS3,his5
CanR,Cyc5
MATE ON YPD PLATE
210/YAUSO
o,URA,TRP,his3,HISS
CanR,Cyc5
FIRST SELECTION OF DIPLOIDS ON-His PLATES
SECOND SELECTION ON -ura,-trp,-ade,lys
210-F10 (300kb):5-B V gcncs
PREPARATION OF DNA PLUGS
ANALYSIS ON CHEF
HYBRIDIZATION WITH A3 PROBE
2-FRAGMENTATION OF 203/210 WITH USING THE VA-A80L FRAGMENT
LIGATED TO pYAC RIGHT ARM:
203/210 (4-8):800 kb
SELECTION ON SORB-URA ADE
203/210-A80L:600kb
Kde IS LOST AFTER THE FRAGMENTATION
A80 (185 kb)
YPH857 (α)
CanS CyhR
y203/210
DIPLOID
Ade+,Ura+,Lys+,Trp+
CanR CyhS
CanS CyhR
MATE
y203/210(600kb)
mpy203/210(a)
CanR CyhS
PLATE ON SC-Ade-Lys-Arg
+CANAVANINE + CYCLOHEXIMIDE
PICK WHITE (Ade+) COLONIES
TEST FOR Trp-,Ura-
SPORULATE
PLATE ON SC+5FOA
PICK WHITE (Ade+) COLONIES
TEST FOR Trp-,Ura-,Lys+,Ade+
(800 kb)

FIG.2

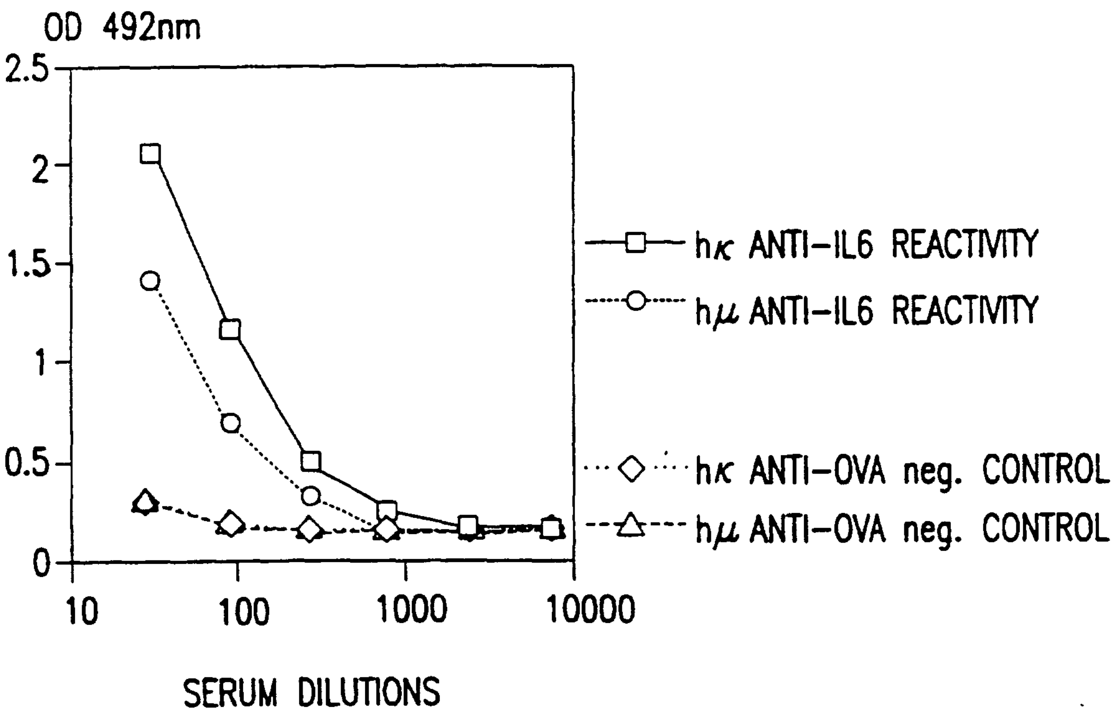
OD 492nm
2.5
2
1.5
1
0.5
0
10
100
1000
10000
SERUM DILUTIONS
hκ ANTI-IL6 REACTIVITY
hμ ANTI-IL6 REACTIVITY
hκ ANTI-OVA neg. CONTROL
hμ ANTI-OVA neg. CONTROL

FIG.3

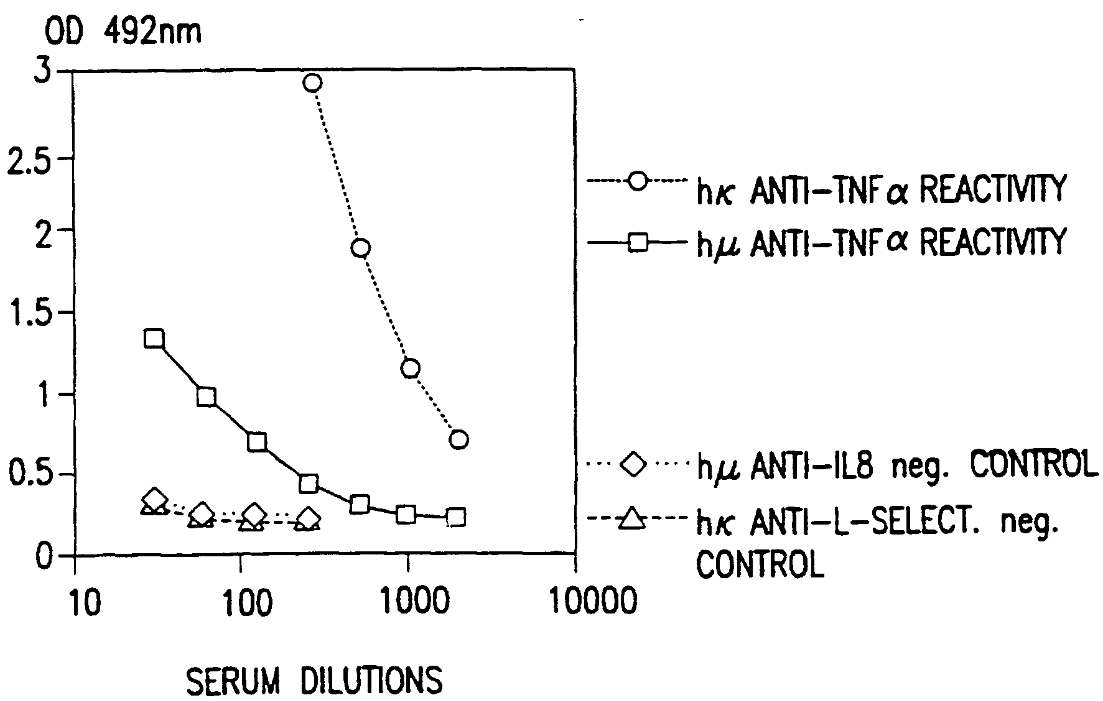
OD 492nm
3
2.5
2
1.5
1
0.5
0
10
100
1000
10000
SERUM DILUTIONS
hκ ANTI-TNFα REACTIVITY
hμ ANTI-TNFα REACTIVITY
hμ ANTI-IL8 neg. CONTROL
hκ ANTI-L-SELECT. neg. CONTROL

FIG.4

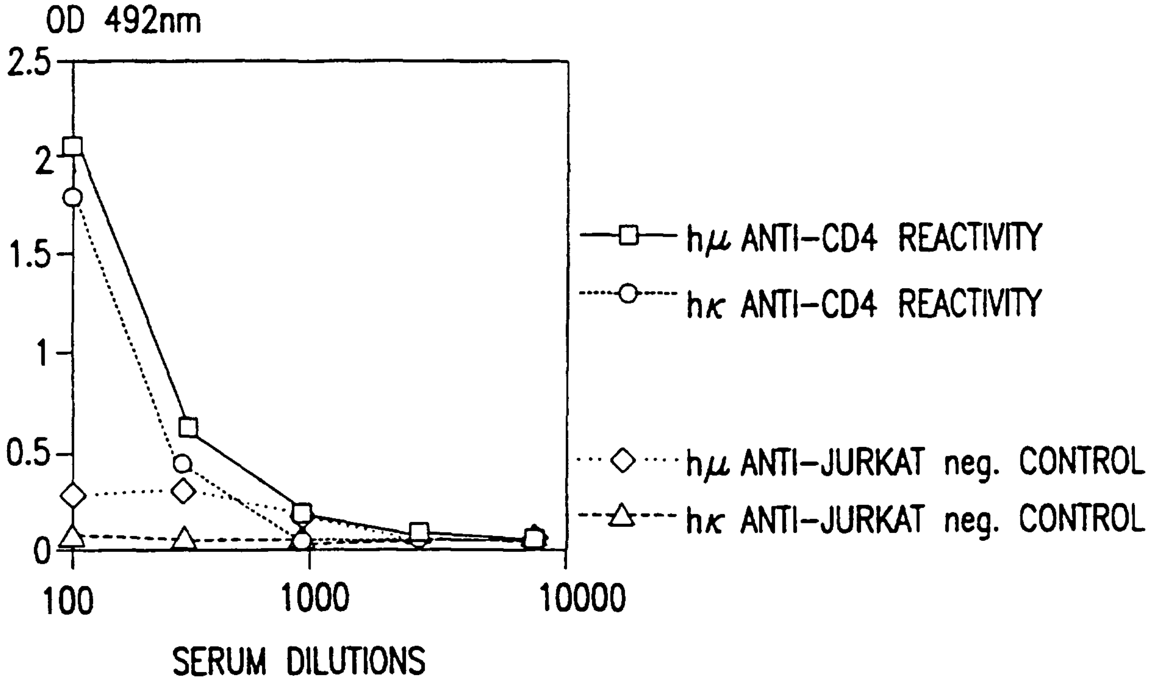
OD 492nm
2.5
2
1.5
1
0.5
0
100
1000
10000
SERUM DILUTIONS
hμ ANTI-CD4 REACTIVITY
hκ ANTI-CD4 REACTIVITY
hμ ANTI-JURKAT neg. CONTROL
hκ ANTI-JURKAT neg. CONTROL

FIG.5

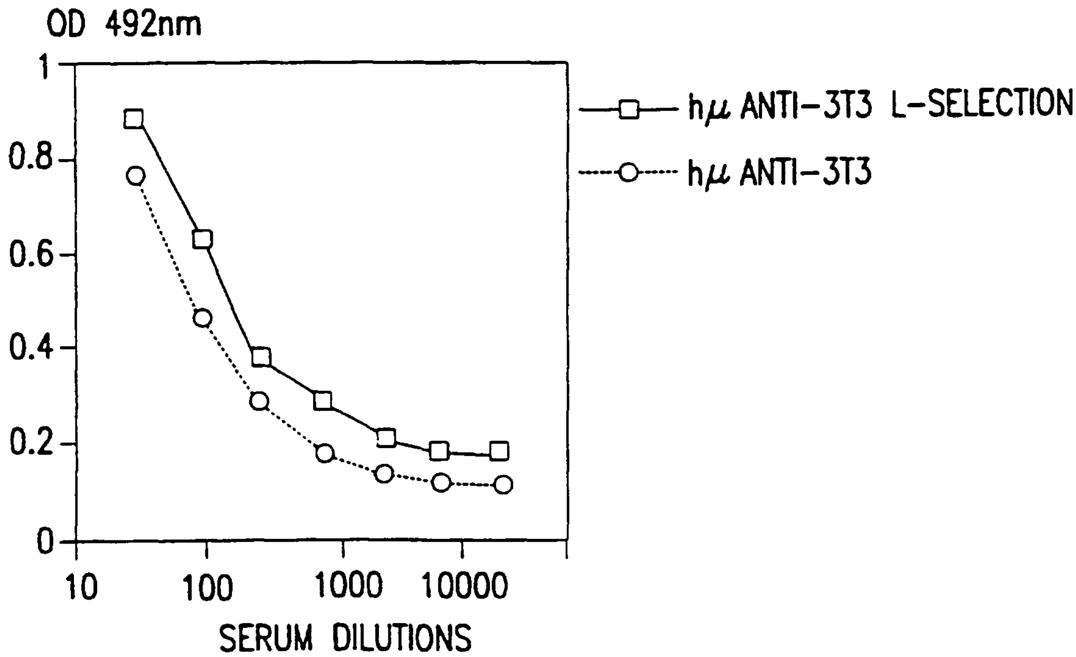
OD 492nm
1
0.8
0.6
0.4
0.2
0
10
100
1000
10000
SERUM DILUTIONS
hμ ANTI-3T3 L-SELECTION
hμ ANTI-3T3

FIG.6

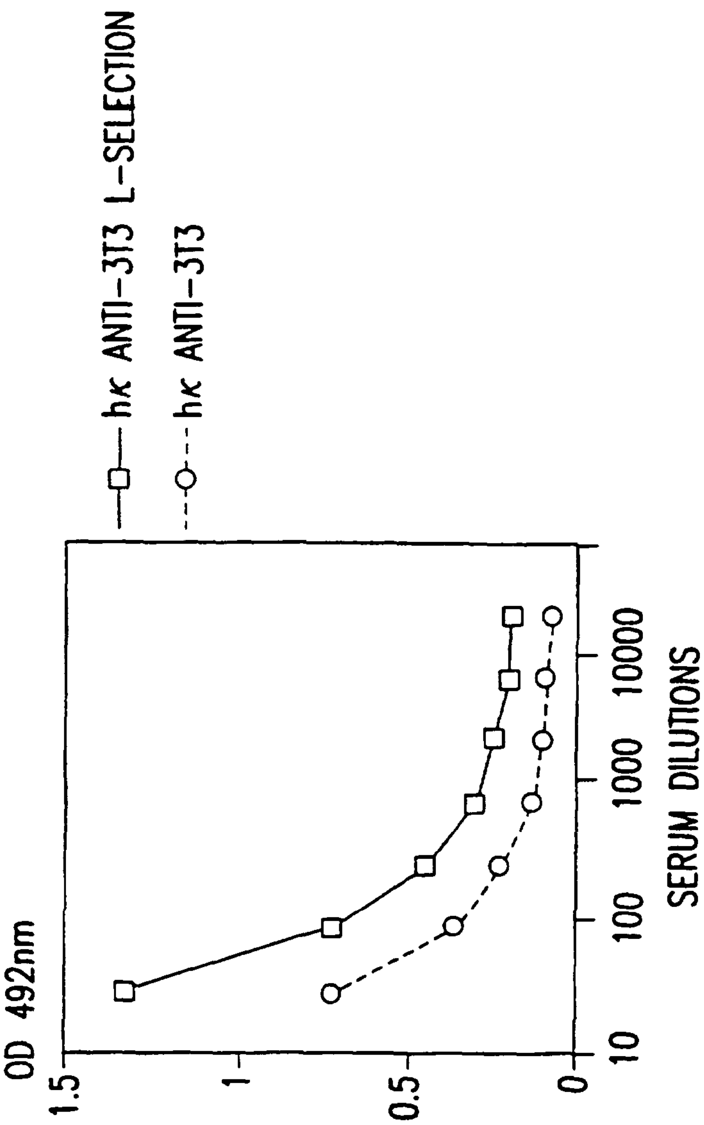
OD 492nm
1.5
1
0.5
0
10
100
1000
10000
SERUM DILUTIONS
hκ ANTI-3T3 L-SELECTION
hκ ANTI-3T3

FIG.7

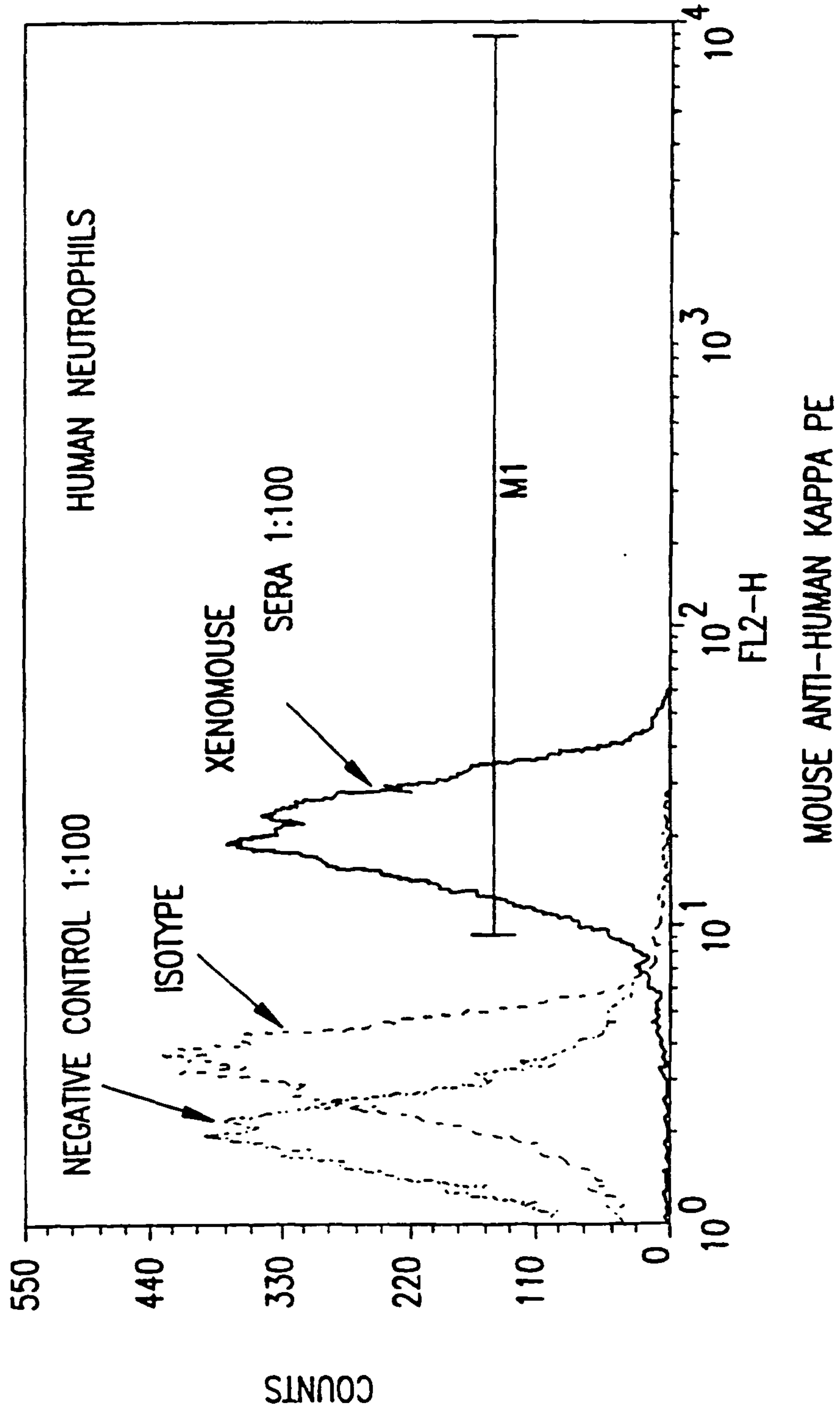
HUMAN NEUTROPHILS
XENOMOUSE
SERA 1:100
NEGATIVE CONTROL 1:100
ISOTYPE
M1
550
440
330
220
110
0
COUNTS
$10^0$
$10^1$
$10^2$
$10^3$
$10^4$
FL2-H
MOUSE ANTI-HUMAN KAPPA PE

FIG.8

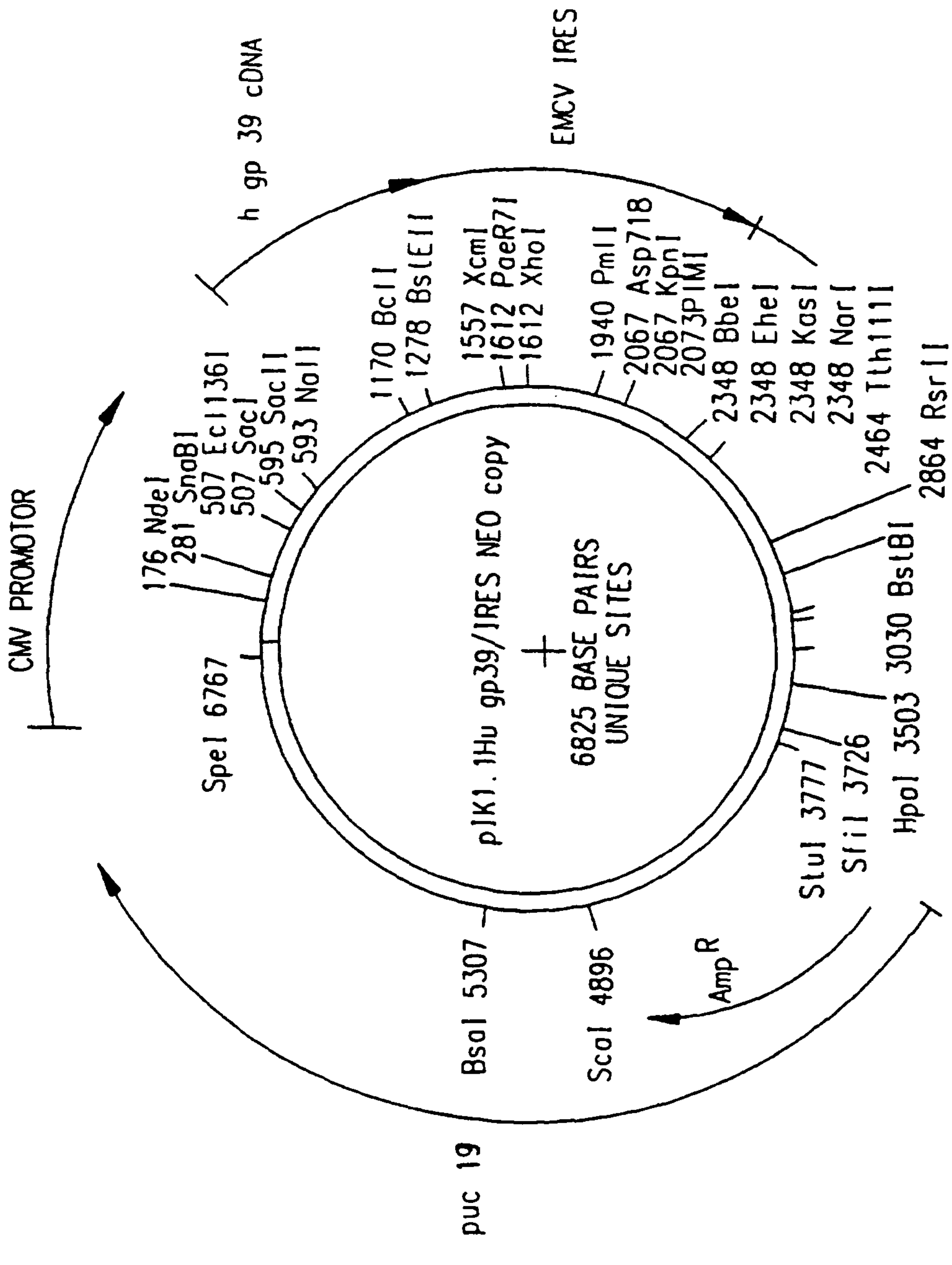
CMV PROMOTOR
h gp 39 cDNA
EMCV IRES
puc 19
Amp$^R$
pJK1.1Hu gp39/IRES NEO copy
6825 BASE PAIRS
UNIQUE SITES
176 NdeI
281 SnaBI
507 Ecl136I
507 SacI
595 SacII
593 NotI
1170 BclI
1278 BstEII
1557 XcmI
1612 PaeR7I
1612 XhoI
1940 PmlI
2067 Asp718
2067 KpnI
2073PlMI
2348 BbeI
2348 EheI
2348 KasI
2348 NarI
2348 Tth111I
2464 RsrII
2864 BstBI
3030 BstBI
HpaI 3503
SfiI 3726
StuI 3777
ScaI 4896
BsaI 5307
SpeI 6767

FIG.9

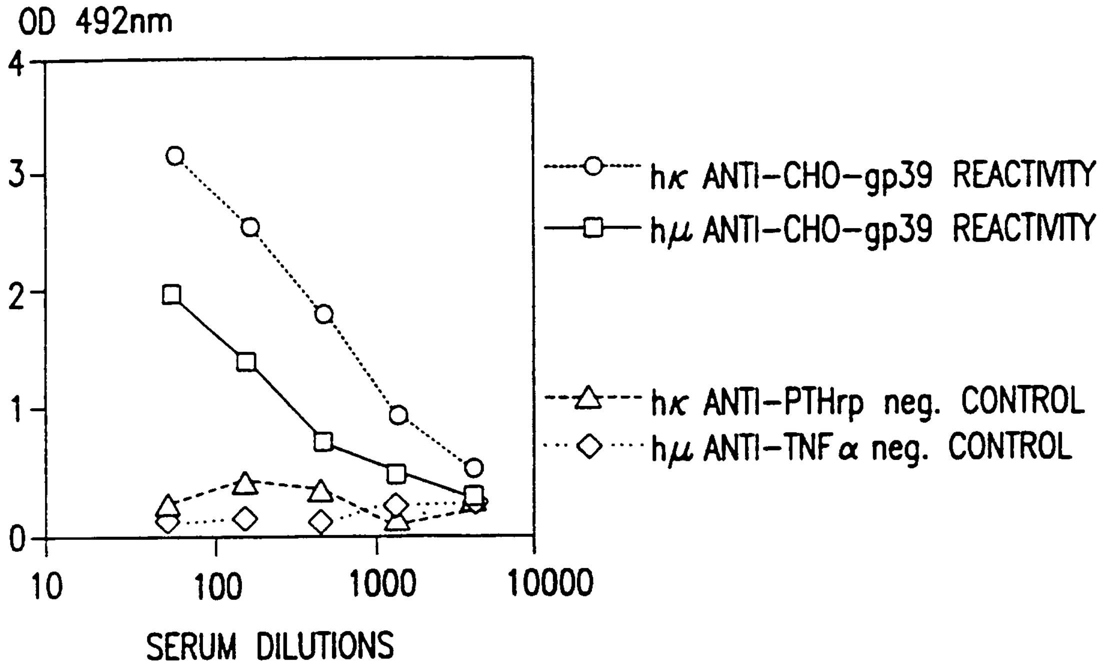
OD 492nm
4
3
2
1
0
10
100
1000
10000
SERUM DILUTIONS
hκ ANTI-CHO-gp39 REACTIVITY
hμ ANTI-CHO-gp39 REACTIVITY
hκ ANTI-PTHrp neg. CONTROL
hμ ANTI-TNFα neg. CONTROL

FIG.10

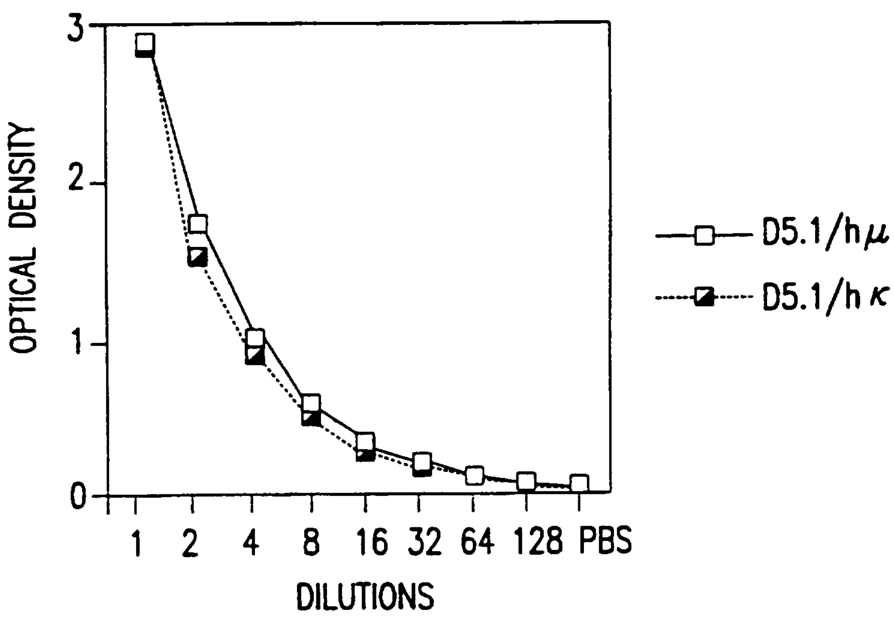
OPTICAL DENSITY
3
2
1
0
1 2 4 8 16 32 64 128 PBS
DILUTIONS
D5.1/hμ
D5.1/hκ

FIG.11

```
                         CDR1
Germline  VH6        AGACCCTCTC ACTCACCTGT GCCATCTCCG GGGACAGTGT CTCTAGCAAC  50
Hybridoma D5. 1.4    AGACCCTCTC ACTCACCTGT GCCATCTCCG GGGACAGTGT CTCTAGCGAC  50
Germline  JH4        ---------- ---------- ---------- ---------- ----------
Germline  D(N1)      ---------- ---------- ---------- ---------- ----------
Germline  hMu        ---------- ---------- ---------- ---------- ----------
                     <------------------------------------------- VH6 -----

                                      CDR2
Germline  VH6        AGTGCTGCTT GGAACTGGAT CAGGCAGTCC CCATCGAGAG GCCTTGAGTG 100
Hybridoma D5. 1.4    AGTGCTGCTT GGAACTGGAT CAGGCAGTCC CCATCGAGAG GCCTTGAGTG 100
Germline  JH4        ---------- ---------- ---------- ---------- ----------
Germline  D(N1)      ---------- ---------- ---------- ---------- ----------
Germline  hMu        ---------- ---------- ---------- ---------- ----------
                     -------------------- VH6 ------------------------->

Germline  VH6        GCTGGGAAGG ACATACTACA GGTCCAAGTG GTATAATGAT TATGCAGTAT 150
Hybridoma D5. 1.4    GCTGGGAAGG ACATACTACA GGTCCAAGTG GTATAATGAT TATGCAGTTT 150
Germline  JH4        ---------- ---------- ---------- ---------- ----------
Germline  D(N1)      ---------- ---------- ---------- ---------- ----------
Germline  hMu        ---------- ---------- ---------- ---------- ----------
                     -------------------- VH6 ------------------------->

Germline  VH6        CTGTGAAAAG TCGAATAACC ATCAACCCAG ACACATCCAA GAACCAGTTC 200
Hybridoma D5. 1.4    CTGTGAAAAG TCGAATAACC ATCAACCCAG ACACATCCAA GAACCAGTTC 200
Germline  JH4        ---------- ---------- ---------- ---------- ----------
Germline  D(N1)      ---------- ---------- ---------- ---------- ----------
Germline  hMu        ---------- ---------- ---------- ---------- ----------
                     -------------------- VH6 ------------------------->

Germline  VH6        TCCCTGCAGC TGAACTCTGT GACTCCCGAG GACACGGCTG TGTATTACTG 250
Hybridoma D5. 1.4    TCCCTGCAGC TGAACTCTGT GACTCCCGAG GACACGGCTG TGTATTACTG 250
Germline  JH4        ---------- ---------- ---------- ---------- ----------
Germline  D(N1)      ---------- ---------- ---------- ---------- ----------
Germline  hMu        ---------- ---------- ---------- ---------- ----------
                     -------------------- VH6 ------------------------->

Germline  VH6        TGCAAGAGA- ---------- ---------- ---------- ---------- 259
Hybridoma D5. 1.4    TGCAAGAGAT ATAGCAGTGG CTGGCGTCCT CTTTGACTGC TGGGGCCAGG 300
Germline  JH4        ---------- ---------- ---------- CTTGACTAGC TGGGGCCAAG  20
Germline  D(N1)      ---------T ATAGCAGCAG CTGG------ ---------- ----------  15
Germline  hMu        ---------- ---------- ---------- ---------- ----------
                     --- VH6 -->| <---- DN1 --|    |<----- JH4 ---------
```

FIG.12A

```
Germline  VH6       ---------- ---------- ---------- ---------- ---------- 259
Hybridoma D5. 1.4   GAACCCTGGT CACCGTCTCC TCAGGGAGTG CATCCGCCCC AACCCTTTTC 350
Germline  JH4       GAACCCTGGT CACCGTCTCC TCA------- ---------- ----------  43
Germline  D(N1)     ---------- ---------- ---------- ---------- ----------  15
Germline  hMu       ---------- ---------- ---GGGAGTG CATCCGCCCC AACCCTTTTC  27
                    -------- JH4 ----------->|<--------- hμ ---------------

Germline  VH6       ---------- ---------- ---------- ---------- ---------- 259
Hybridoma D5. 1.4   CCCCTCGTCT CCTGTGAGAA TTCCCCGTCG GATACGAGCA GCGTGGCCGT 400
Germline  JH4       ---------- ---------- ---------- ---------- ----------  43
Germline  D(N1)     ---------- ---------- ---------- ---------- ----------  15
Germline  hMu       CCCCTCGTCT CCTGTGAGAA TTCCCCGTCG GATACGAGCA GCGTGGCCGT  77
                    ---------------------- hμ ---------------------------->
```

FIG.12B

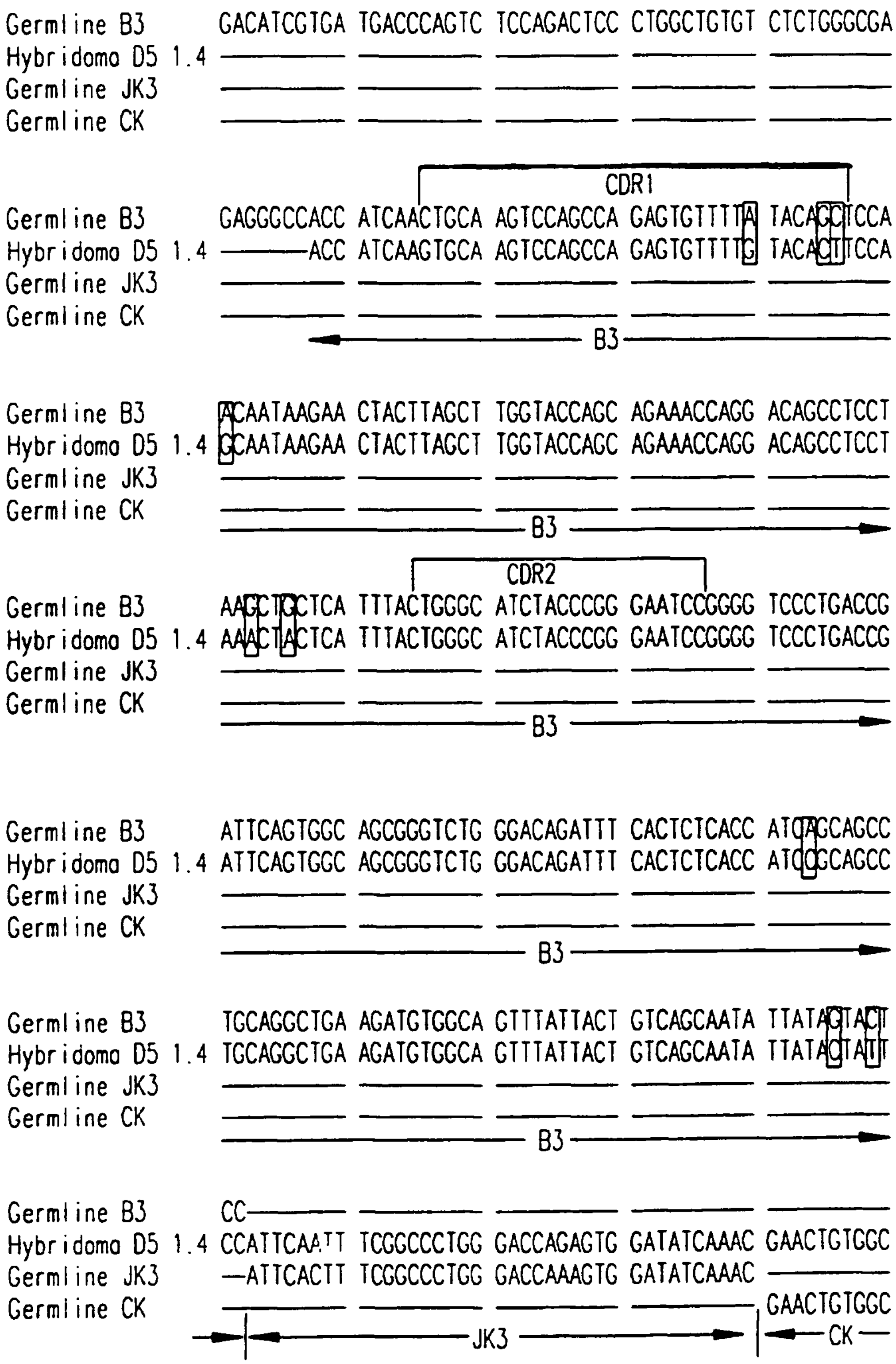
Germline B3 GACATCGTGA TGACCCAGTC TCCAGACTCC CTGGCTGTGT CTCTGGGCGA
Hybridoma D5 1.4
Germline JK3
Germline CK
CDR1
Germline B3 GAGGGCCACC ATCAACTGCA AGTCCAGCCA GAGTGTTTTA TACAGCTCCA
Hybridoma D5 1.4 ACC ATCAAGTGCA AGTCCAGCCA GAGTGTTTTG TACACTTCCA
Germline JK3
Germline CK
B3
Germline B3 ACAATAAGAA CTACTTAGCT TGGTACCAGC AGAAACCAGG ACAGCCTCCT
Hybridoma D5 1.4 GCAATAAGAA CTACTTAGCT TGGTACCAGC AGAAACCAGG ACAGCCTCCT
Germline JK3
Germline CK
B3
CDR2
Germline B3 AAGCTGCTCA TTTACTGGGC ATCTACCCGG GAATCCGGGG TCCCTGACCG
Hybridoma D5 1.4 AAACTACTCA TTTACTGGGC ATCTACCCGG GAATCCGGGG TCCCTGACCG
Germline JK3
Germline CK
B3
Germline B3 ATTCAGTGGC AGCGGGTCTG GGACAGATTT CACTCTCACC ATCAGCAGCC
Hybridoma D5 1.4 ATTCAGTGGC AGCGGGTCTG GGACAGATTT CACTCTCACC ATCGGCAGCC
Germline JK3
Germline CK
B3
Germline B3 TGCAGGCTGA AGATGTGGCA GTTTATTACT GTCAGCAATA TTATAGTACT
Hybridoma D5 1.4 TGCAGGCTGA AGATGTGGCA GTTTATTACT GTCAGCAATA TTATACTATT
Germline JK3
Germline CK
B3
Germline B3 CC
Hybridoma D5 1.4 CCATTCAATT TCGGCCCTGG GACCAGAGTG GATATCAAAC GAACTGTGGC
Germline JK3 ATTCACTT TCGGCCCTGG GACCAAAGTG GATATCAAAC
Germline CK GAACTGTGGC
JK3
CK

FIG.13A

```
Germline B3          ---------- ---------- ---------- ---------- ----------
Hybridoma D5 1.4     TGCACCATCT GTCTTCATCT TCCCGCCATC TGATGAGCAG TTGAAATCTG
Germline JK3         ---------- ---------- ---------- ---------- ----------
Germline CK          TGCACCATCT GTCTTCATCT TCCCGCCATC TGATGAGCAG TTGAAATCTG
                     ------------------------- CK ------------------------>

Germline B3          ---------- ---------- ---------- ---------- ----------
Hybridoma D5 1.4     GAACTGCCTC TGTTGTGTGC CTGCTGAATA ACTTCTATCC CAGAGAGGCC
Germline JK3         ---------- ---------- ---------- ---------- ----------
Germline CK          GAACTGCCTC TGTTGTGTGC CTGCTGAATA ACTTCTATCC CAGAGAGGCC
                     ------------------------- CK ------------------------>

Germline B3          ---------- ---------- ---------- ---------- ----------
Hybridoma D5 1.4     AAAGTACAGT GGAAGGTGGA TAACGCCCTC CAATCGGGTT GGGGAAAAA
Germline JK3         ---------- ---------- ---------- ---------- ----------
Germline CK          AAAGTACAGT GGAAGGTGGA TAACGCCCTC CAATCGGGT- ----------
                     ------------------------- CK ------------------------>
```

FIG.13B

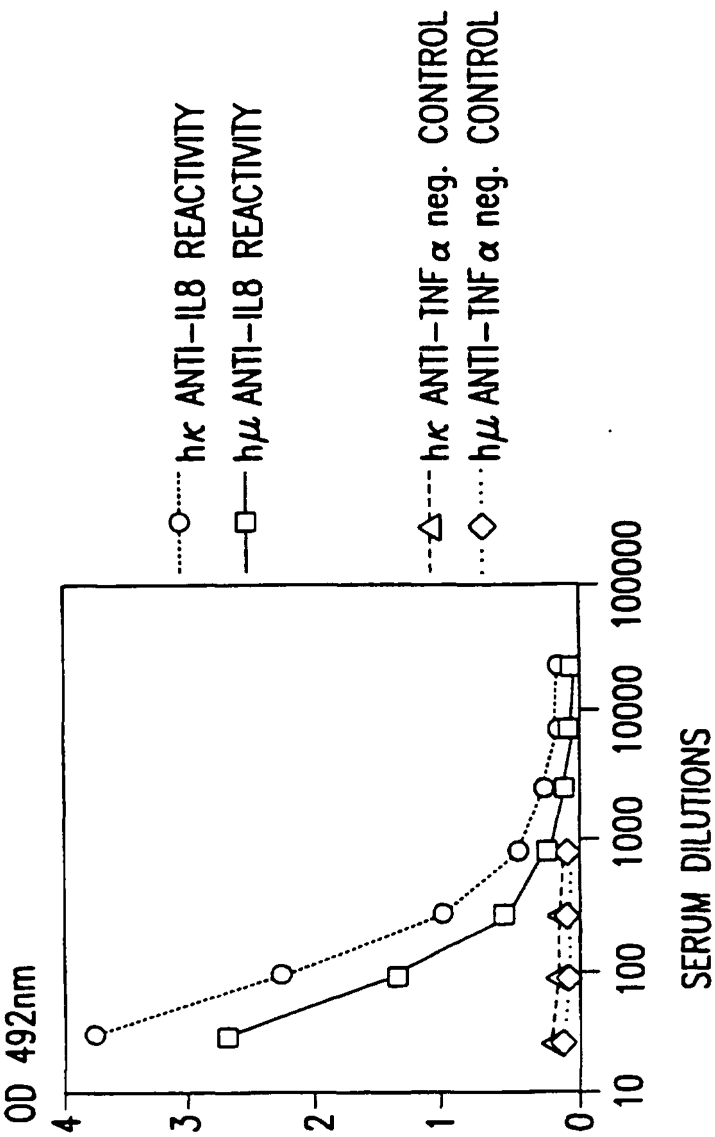
OD 492nm
4
3
2
1
0
10
100
1000
10000
100000
SERUM DILUTIONS
hκ ANTI-IL8 REACTIVITY
hμ ANTI-IL8 REACTIVITY
hκ ANTI-TNFα neg. CONTROL
hμ ANTI-TNFα neg. CONTROL

FIG.14

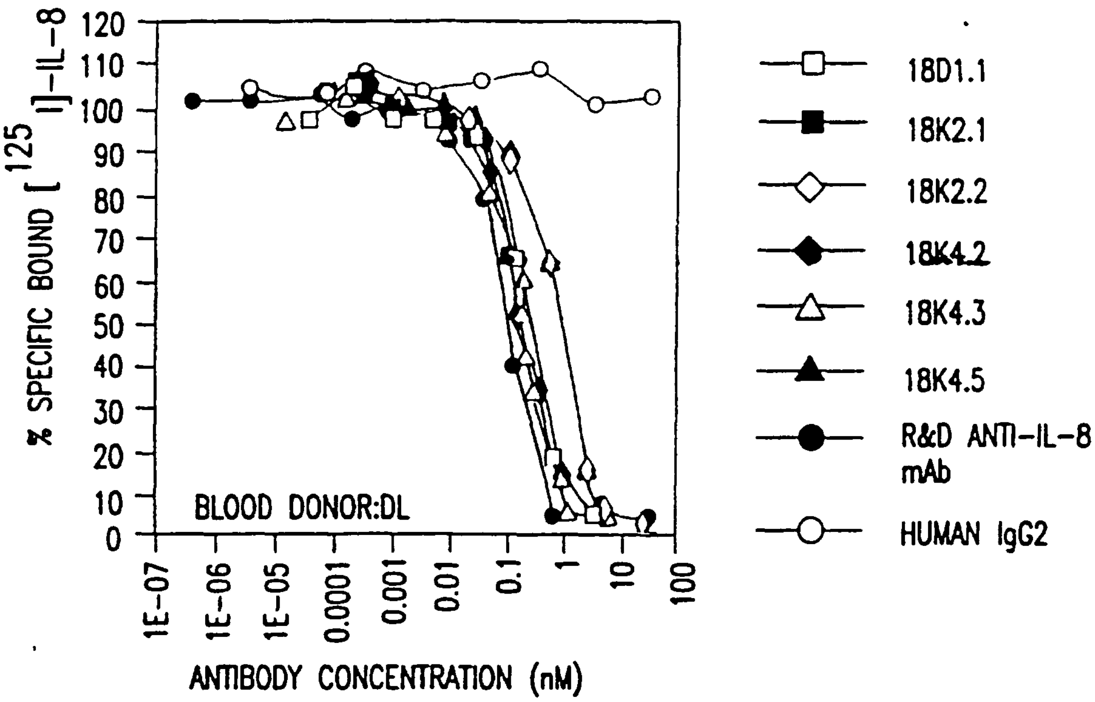


FIG.15A

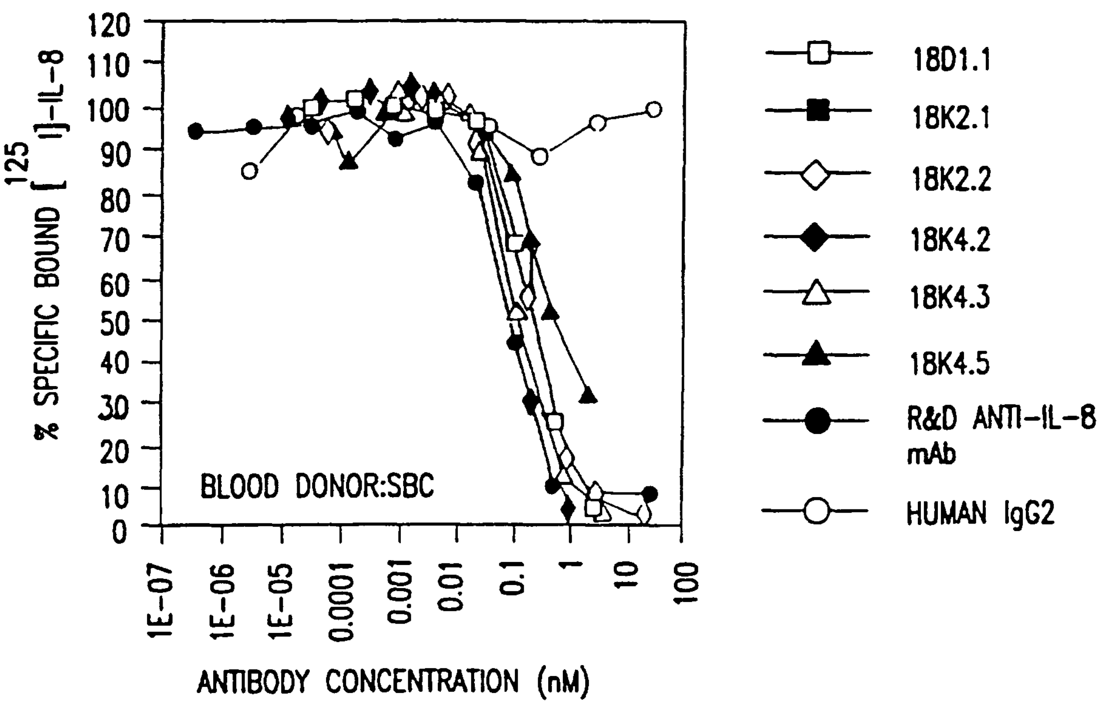


FIG.15B

[CCTGTCCCTCACCTGCGCTGTCTATGGTGGGTCCTTCAGTGGTTACTACTGGAGCTGGATCCGCC
AGCCCCCAGGGAAGGGACTGGAGTGGATTGGGGAAATCAATCAAAGTGGAAGCACCAATTACAA
CCCGTCCCTCAAGAGTCGAGTCATCATATCAATAGACACGTCCAAGACCCAGTTCTCCCTGAAGT
TGAGCTCTGTGACCGCCGCGGACACGGCTGTGTATTACTGTGCGAGAGA][GACTCCCC][ATGCT
TTTGATATCTGGGGCCAAGGGACAATGGTCACCGTCTCTTCAG]CCTCCACCAAGGGCCCATCGG
TCTTCCCCCTGGCGCCCTGCTCCAGGAGCACCTCCGAGAGCACAGC(GC)GCCCTGGGCTGCCTG
GTCAAGGACTACTTCC

FIG. 16A

[CAGTCTCCATCCTCCCTGTCTGCATCTGTAGGCGACAGAGTCACCATCACTTGCCAGGCGAGTC
AGGACATTAGTAAGTTTTTAAGTTGGTTTCAACAGAAACCAGGGAAAGCCCCTAAACTCCTGATC
TACGGTACATCCTATTTGGAAACCGGGGTCCCATCAAGTTTCAGTGGAAGTGGATCTGGGACAGA
TTTTACTCTCACCATCAGCAGCCTGCAGCCTGAAGATGTTGCAACATATTTCTGTAACAGNATG
ATGATCTCCC][ATACACTTTCGGCCCTGGGACCAAAGTGGATATCAAAC]GAACTGTGGCTGCAC
CATCTGTCTTCATCTTCCCGCCATCTGATGAGCAGTTGAAATCTGGAACTGCCTCTGTTGTGTGCC
TGCTGAATAACTTCTATCCCAGAGAGGCCAAAGTACAGTGGAAGGTGGATAACGCCC

FIG. 16B

[AGGTCCCTGAGACTCTCCTGTGCAGCCTCTGGATTCACCTTCAGTAGCTATGGCATGCACTGGNT
CCGCCAGGCTCCAGGCAAGGGGCTGGAGTGGGTGGCAGAAATATCATATGATGGAAGTAATAAA
TACTATGTAGACTCCGTGAAGGGCCGACTCACCATCTCCAGAGACAATTCCAAGAACACGCTGT
ATCTGCAAATGAACAGCCTGAGAGCTGAGGACACGGCTGTGTATTACTGTGCGAGAGA][CCGAC
TGGGGAT][CTTTGACTACTGGGGCCAGGGAACCCTGGTCACCGTCTCCTCAG]CCTCCACCAAGG
GCCCATCGGTCTTCCCCCTGGCGCCCTGCTCCAGGAGCACCTCCGAGAGCACAGC(GC)GGCCCT
GGGCTGCCTGGTCCAAGGACTACTTCCCCCGAACCGGTGACGGTGTCGTGGAACTCAGGCGCTC
TGACCAG

FIG. 16C

[CTGACNCAGTCTCCAGACTCCCTGGCTGTGTCTCTGGGCGAGAGGGCCACCATCAACTGCAAGT
CCAGCCAGAGTGTTTTATACATCTCCAACAATAAGAACTACTTAGCTTGGTACCAGCAGAAACCA
GGACAGTCTCCTAAACTGCTCATTTACTGGGCATCTACCCGGAAATCCGGGGTCCCTGACCGATT
CAGTGGCAGCGGGTCTGGGACAGATTTCACTCTCACCATCAGCAGCCTGCAGGCTGAAGATGTG
GCAGTTTATTACTGTCAACAGTATTATGATACTCC][ATTCACTTTCGGCCCTGGGACCAAAGTGG
ATATCAAAC]GAACTGTGGCTGCACCATCTGTCTTCATCTTCCCGCCATCTGATGAGCAGTTGAAA
TCTGGAACTGCCTCTGTTGTGTGCCTGCTGAATAACTTCTATCCCAGAGAGGCCAAAGTACAGTG
GAAGGTGGNTAACGCCCCA

FIG. 16D

[TCCCTCACCTGCGCTGTCTATGGTGGGTCCTTCAGTGGTTACTACTGGACCTGGATCCGCCAGCC
CCCAGGGAAGGGGCTGGAGTGGATTGGGGAAATCATTCATCATGGAAACACCAACTACAACCCG
TCCCTCAAGAGTCGAGTCTCCATATCAGTTGACACGTCCAAGAACCAGTTCTCCCTGACACTGAG
CTCTGTGACCGCCGCGGACACGGCTGTGTATTACTGTGCGAGAGG][GGGAGCAGTGGCTGCG][T
TTGACTACTGGGGCCAGGGAACCCTGGTCACCGTCTCCTCAG]CCTCCACCAAGGGCCCATCGGT
CTTCCCCCTGGCGCCCTGCTCCAGGAGCACCTCCGAGAGCACAGC(GC)GGCCCTGGGCTGCCTG
GTCAAGGACTACTTCCCCCGAACCGGTGACGGTGTCGTGGAACTCAGGCGCTCTGACCAGCGGC
GTGCACACCTTCCCA

FIG. 16E

[TGACCCAGTCTCCATCCTCCCTGTCTGCATCTGTAGGAGACAGAGTCACCATCACTTGCCAGGC
GAGTCAGGACATTAGTAACTATTTAAATTGGTATCAACAGAAAGCAGGGAAAGCCCCTAAGGTCC
TGATCTACGCTGCATCCAATTTGGAAGCAGGGGTCCCATCAAGGTTCAGTGGAAGTGGATCTGGG
ACAGATTTTACTTTCACCATCAGCAGCCTGCAGCCTGAAGATATTGCAACATATTATTGTCAACA
CTATGATAATCT]A[CTCACTTTCGGCGGAGGGACCAAGGTAGAGATCAAAC]GAACTGTGGCTGC
ACCATCTGTCTTCATCTTCCCGCCATCTGATGAGCAGTTGAAATCTGGACTGCCTCTGTTGTGTG
CCTGCTGAATAACTTCTATCCCAGAGAGGCCAAAGTACAGTGGAAGGTGG

FIG. 16F

AGTCTCTGAAGATCTCCTGTAAGGGTTCTGGATACAGCTTTACCAGCTACTGGATCGGCTGGGTG
CGCCAGATGCCCGGGAAAGGCCTGGAGTGGATGGGGATCATCTATCCTGGTGACTCTGATACCA
GATACAGCCCGTCCTTCCAAGGCCAGGTCACCATCTCAGCCGACAAGTCCATCAGCACCGCCTA
CCTGCAGTGGAGCAGCCTGAAGGCCTCGGACACCGCCATGTATTACTGTGCGAGACA][GGACGG
TG][ACTCCTTTGACTACTGGGGCCAGGGAACCCTGGTCACCGTCTCCTCAG]CCTCCACCAAGGG
CCCATCGGTCTTCCCCCTGGCGCCCTGCTCCAGGAGCACCTCCGAGAGCACAGC(GC)GGCCCTG
GGCTGCCTGGTCCAAGGACTACTTCCCCCGAACCGGTGACGGTGTCGTGGAACTCAGGCGCTCT
GACCAGCGGCGTGCACACCTTCCCACTGCCA

FIG. 16G

TGTCTGCATCTATTGGAGACAGAGTCACCATCACTTGCCGGGCAAGTCAGAGCATTAGCAACTA
TTTAAATTGGTATCAGCAGAAACCAGGGAAAGCCCCTAAGTTCCTGATCTATGGTGCATCCAGT
TTGGAAAGTGGGGTCCCATCANGGTTCAGTGGCAGTGGATCTGGGACAGATTTCACTCTCACCAT
CAGCAGCCTGCAACCTGNGGATTTTGCAACTTACTACTGTCAACAGAGTTACAGTAACCC]T[CTC
ACTTTCGGCGGNGGGACCAANGTGGAGATCAAAC]GAACTGTGGCTGCACCATCTGTCTTCATCT
TCCCGCCATCTGATGAGCAGTTGAAATCTGGAACTGCCTCTGTTGTGTGCCTGCTGAATAACTTCT
ATCCCAGAGAGGCCAAAGTACA

FIG. 16H

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9402602 A
- WO 9204372 A
- EP 37734 A
- EP 05028220 A
- EP 96913247 A
- US 9605928 W
- US 08430938 B


### Non-patent literature cited in the description


- **GREEN et al.** *Nature Genetics,* 1994, vol. 7, 13-21
- **CALBERTSEN et al.** *PNAS,* 1990, vol. 87, 4256
- **MENDEZ et al.** *Genomics,* 1995, vol. 26, 294-307
- **BURKE et al.** *Science,* 1987, vol. 236, 806-812
- **BROWNSTEIN et al.** *Science,* 1989, vol. 244, 1348-1351
- **BURKE et al.** *Methods in Enzymology,* 1991, vol. 194, 251-270
- **HUSTON et al.** *Methods in Enzymology,* 1991, vol. 203, 46-63
- **TRAUNECKER, A. et al.** *Int. J. Cancer,* 1992, vol. 7, 51-52
- **HIGGINS, P.J. et al.** *J. Infect Disease,* 1992, vol. 166, 198-202
- **FANGER, M.W. et al.** *Cancer Treat Res,* 1993, vol. 68, 181-194
- **FANGER, M.W. et al.** *Crit Rev Immunol,* 1992, vol. 12, 101-124
- **BYERS, B.S. et al.** *Seminars Cell Biol,* 1991, vol. 2, 59-70
- **FANGER, M.W. et al.** *Immunol Today,* 1991, vol. 12, 51-54
- **KOHLER ; MILSTEIN.** *Nature,* 1975, vol. 245, 495
- **GRIFFITHS, A.D. et al.** *EMBO J,* 1994, vol. 13, 3245-3260
- **NISSIM, A. et al.** *EMBO. J,* 692-698
- **GRIFFITHS, A.D. et al.** *EMBO. J,* vol. 12, 725-734
- **BURTON, D.R. et al.** *Proc. Natl. Acad. Sci. USA,* 1991, vol. 88, 10134-10137
- **ZEBEDEE, S.L. et al.** *Proc. Natl. Acad. Sci. USA,* 1992, vol. 89, 3175-3179
- **BARBAS III, C.F. et al.** *Proc. Natl. Acad. Sci. USA,* 1991, vol. 89, 10164-20168
- **BARBAS III C.F. et al.** *Proc. Natl. Acad. Sci. USA,* 1991, vol. 89, 4457-4461
- **CRAMERI, A. et al.** *BioTechniques,* 1995, vol. 88, 194-196
- **RAPOPORT, B. et al.** *Immunol. Today,* 1995, vol. 16, 43-49
- **PORTOLANO, S. et al.** *J. Immunol.,* 1993, vol. 151, 2839-2851
- **VOGEL, M. et al.** *Eur J. Immunol.,* 1994, vol. 24, 1200-1207
- **GRIFFITHS, A.D. et al.** *EMBO J.,* 1994, vol. 13, 3245-3260
- **GRIFFITHS, A.D. et al.** *EMBO J.,* 1993, vol. 12, 725-734
- **PERSSON, M.A.A. et al.** *Proc. Natl. Acad. Sci. USA,* 1991, vol. 88, 2432-2436
- **GRIFFITHS, A.D.** *Curr. Opin. Immunol.,* 1993, vol. 5, 263-267
- **HOOGENBOOM, H.R. et al.** *J. Mol. Biol.,* 1992, vol. 227, 381-388
- **LERNER, R.A. et al.** *Science,* 1992, vol. 258, 1313-1314
- **NISSIM A. et al.** *EMBO J.,* 1994, vol. 13, 692-698
- **MARKS, J.D. et al.** *J. Mol. Biol.,* 1991, 581-596
- **WINTER, G. et al.** *Annu. Rev. Immunol.,* 1994, vol. 12, 433-55
- **BARBAS III, C.F. et al.** *Proc. Natl. Acad. Sci. USA,* 1991, vol. 88, 7978-7982
- **MOORE et al.** *Nature,* 14 October 1993, vol. 365
- **ROBERTS et al.** *Blood,* 1994, vol. 84, 2878
- **CALLAN, M. et al.** *Proc Natl Acad Sci USA,* 1993, vol. 90, 10454
- **FINER et al.** *Blood,* 1994, vol. 83, 43
- **TEDDER et al.** *J. Immunol,* 1990, vol. 144, 532
- **SUBRANANI et al.** *Mol Cell Biol,* 1981, vol. 9, 854
- **GALFRE, G. ; MILSTEIN, C.** *Methods in Enzymology,* 1981, vol. 73, 3-46

- **RATCLIFFE et al.** *J. Immunol. Methods,* 1990, vol. 127, 109
- **KARLSSON et al.** Kinetic analysis of monoclonal antibody-antigen interaction with a new biosensor based analytical system. *J. Immunol. Methods,* vol. 145, 229
- **MARKS.** *Euro J. Immunol,* 1991, vol. 21, 985-991